# EUROPEAN PATENT SPECIFICATION

(11) **EP 3 359 513 B1**
(45) Date of publication and mention of the grant of the patent: **30.12.2020**
(21) Application number: 16853206.7
(22) Date of filing: 06.10.2016
(51) Int. Cl.: C07B 39/00, C07C 17/363, C07C 22/04, C07C 25/02, C07C 23/10, C07C 201/12, C07C 205/08, C07C 205/12, C07C 205/37, C07C 25/06, C07C 25/08, C07C 25/10, C07C 25/13, C07C 253/30, C07C 255/50, C07D 307/89, C07C 19/01

(54) **PROCESS FOR THE PREPARATION OF ORGANIC HALIDES**
VERFAHREN ZUR HERSTELLUNG VON ORGANISCHEN HALIDEN
PROCÉDÉ DE PRÉPARATION D'HALOGÉNURES ORGANIQUES

(30) Priority: 07.10.2015 US 201562238208 P
(43) Date of publication of application: 15.08.2018
(73) Proprietor: Technion Research & Development Foundation Limited, Technion City, Haifa 3200004 (IL)
(72) Inventor: GANDELMAN, Mark, 4226019 Kfar-Saba (IL); NISNEVICH, Gennady A., 3299210 Haifa (IL); KULBITSKI, Kseniya, 324935 Haifa (IL); ARTARYAN, Alexander, 3200008 Haifa (IL)
(74) Representative: Pearl Cohen Zedek Latzer Baratz UK LLP
(86) International application number: PCT/IL2016/051084
(87) International publication number: WO 2017/060906

(56) References cited:
- WO-A1-2011/154953
- IN-A- 803D EL1 999
- LEONARDO R. SODRÉ ET AL: "A Green Hunsdiecker Reaction of Cinnamic Acids", JOURNAL OF THE BRAZILIAN CHEMICAL SOCIETY, vol. 24, no. 2, 1 January 2013 (2013-01-01), pages 212-218, XP55572808, São Paulo; BR ISSN: 0103-5053, DOI: 10.5935/0103-5053.20130027
- D. NASKAR; ET AL.: '1-Haloalkynes from Propiolic Acids: A Novel Catalytic Halodecarboxylation Protocol' JOURNAL OF ORGANIC CHEMISTRY vol. 64, 08 October 1999, ISSN 0022-3263 pages 6896 - 6897, XP002656405
- S. CHOWDHURY; ET AL.: 'The First Example of a Catalytic Hunsdiecker Reaction: Synthesis of beta-Halostyrenes' JOURNAL OF ORGANIC CHEMISTRY vol. 62, 01 January 1997, ISSN 0022-3263 pages 199 - 200, XP002656406
- D. NASKAR; ET AL.: 'Catalytic Hunsdiecker Reaction and One-Pot Catalytic Hunsdiecker- Heck Strategy: Synthesis of alpha,beta-Unsaturated Aromatic Halides, alpha-(Dihalomethyl) benzenemethanols, 5-Aryl-2,4-pentadienoic acids, Dienoates and Dienamides' TETRAHDRON vol. 56, 2000, ISSN 0040-4020 pages 1369 - 1377, XP002656408
- F.G. BORDWELL ET AL.: 'Synthesis of Dihalomethyl and alpha-Haloalkyl Sulfones by the Halogenative Decarboxylation of alpha-Aryl- and alpha-Alkylsulfonylakanecarboxylic Acids' JOURNAL OF ORGANIC CHEMISTRY vol. 39, 01 January 1974, ISSN 0022-3263 pages 2516 - 2519, XP002656409

## Description

### FIELD OF THE INVENTION

The present invention provides a halo-de-carboxylation process for the preparation of organic chlorides, organic bromides and mixtures thereof, from their corresponding carboxylic acids, using a chlorinating agent selected from trichloroisocyanuric acid (TCCA), dichloroisocyanuric acid (DCCA), or any combination thereof, and a brominating agent. The invention further provides a composition comprising a carboxylic acid, brominating agent and chlorinating agent selected from trichloroisocyanuric acid (TCCA), dichloroisocyanuric acid (DCCA), or combination thereof.

### BACKGROUND OF THE INVENTION

Organic bromides and chlorides are stable compounds which are used commercially for many applications, such as pharmaceuticals, agriculture, disinfectants, flame extinguishing agents, and dyes. The organic halides have found wide use in numerous industrial applications as chemical intermediates for the production of other commercial organic compounds. (Ullmann's Encyclopedia of Industrial Chemistry 2012, v. 6, 331-358; v. 8, 483-519).

Carboxylic acids are widely available and cheap raw materials in the organic synthesis. Therefore, the oxidative decarboxylation of carboxylic acids with concomitant replacement by halogen (halo-de-carboxylation) is an extremely useful alternative for regioselective syntheses of organic halides.

The Hunsdiecker reaction (Tetrahedron 1971, v. 27, 5323) is a halo-de-carboxylation reaction, which utilizes treatment of anhydrous silver salt of organic carboxylic acid with molecular bromine or chlorine in an inert solvent (Tetrahedron 1971, v. 27, 5323). However, the reaction is extremely sensitive to presence of trace amounts of water, which lead to the recovery of unreacted acid. Another way to perform the Hunsdiecker reaction is by using a mixture of organic carboxylic acid and Br₂/HgO (J. Org. Chem. 1965, v. 30, 415) instead of the silver salt.

Accordingly, the Hunsdiecker reaction and/or its modifications, use heavy metal salts such as those of silver and mercury, therefore the disadvantages of such procedures for the pharmaceutical industry are obvious.

The Barton halo-de-carboxylation procedure (Barton et al., Tetrahedron 1985, v. 41, 3901; 1987, v. 43, 4321) is directed to the conversion of organic carboxylic acids to the esters of *N*-hydroxypyridine-2-thione. The thiohydroxamic esters are halogenated by XCCl₃, wherein X is Cl or Br. Thiopyridines are formed in the reaction as co-products.

Additional process for converting carboxylic acids to their corresponding bromides is by treating the carboxylic acid with (diacetoxyiodo)benzene and bromine or LiBr as bromine source (Tetrahedron 2000, v. 56, 2703; Synlett 2011, 1563). However, in this reaction, it is difficult to separate the desired product from iodobenzene, which is formed as co-product in the reaction.

A bromo- and chlorodecarboxylation of aromatic carboxylic acids using CuBr₂ and CuCl₂ as the halogen sources has been developed by Wu et. al. (Tetrahedron Letters 2010, v. 51, 6646) and Liu et. al. (Tetrahedron Letters 2013, v. 54, 3079), which also utilize the use of heavy metals in their reactions.

Another example for bromodecarboxylation utilizes the reagent system I₂O₅-KBr for bromodecarboxylation of electron-rich arenecarboxylic acids (Synlett 2014, v. 25, 2508). This method, however, is limited to preparation of specific brominated phenol ether derivatives. WO 2011/154953 discloses a process for the preparation of high yield alkyl or aryl iodide from its corresponding carboxylic acid using N-iodo amides.

N-Bromo and N-chloro amides such as *N*-bromosuccinimide (Chem. Pharm. Bull. 2002, v. 50, 941), 1,3-dibromo-5,5-dimethylhydantoin (Bioorg. Med. Chem. 2008, v. 16, 10001; Bioorg. Med. Chem. Lett. 2011, v. 21, 3227; Tetrahedron 2014, v. 70, 318), dibromoisocyanuric acid (Monatsh. Chem. 1968, v. 99, 815; 1969, v. 100, 42 & 1977, v. 108, 1067), tribromoisocyanuric acid (Synlett 2013, v. 24, 603), trichloroisocyanuric acid (TCCA) (JOC 1970, v. 35, 719) *N*-chlorosuccinimide (NCS) (ACS Med. Chem, Lett. 2010, v. 1, 30; Chem. Pharm. Bull. 2002, v. 50, 941; Org. Proc. R&D 2010, 14, 1254) are useful reagents for the electrophilic halogenation of aromatic carboxylic acids in the *meta*-position with respect to the carboxylic group. However, the use of these reagents in halo-de-carboxylation reactions is limited.

For example, reaction of N-bromosuccinimide with arenecarboxylic acids, predominantly electron-rich arenecarboxylic acids, yields bromoarenenes (*IN803DEL1999;* JOC 2009, v. 74, 8874; Tetrahedron Lett. 2007, v. 48, 5429). Reaction of aryl acrylic and propiolic acids with N-halosuccinimides (JOC 2002, v. 67, 7861) and trihaloisocyanuric acids (J. Braz. Chem. Soc. 2013, v. 24, 213) yields aryl vinyl and ethynyl halides. All of these reactions are heterolytic reactions that do not require initiation with radical initiators or UV-visible light irradiation.

The conversion of carboxylic acid, R-CO₂H, to their corresponding halide, R-X, is therefore a rather difficult transformation. There is a need for the development of new strategies for halo-de-carboxylation.

### SUMMARY OF THE INVENTION

In one embodiment, this invention is directed to a process for the preparation of organic halide of formula (**1A**) from a carboxylic acid of formula **(2A)** represented by scheme 1: 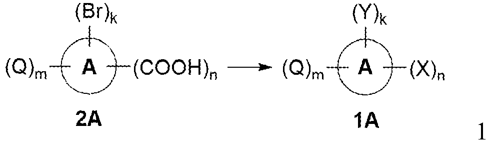 said process comprises radical halo-de-carboxylation reaction comprising reacting carboxylic acid **(2A)** with a chloroisocyanurate and a brominating agent to yield organic halide (**1A**);
wherein
said chloroisocyanurate is trichloroisocyanuric acid, dichloroisocyanuric acid, or any combination thereof;
**A** is arene, alkane, cycloalkane or saturated heterocycle;
**n** is an integer greater than or equal to 1;
**X** is Cl or Br; wherein if n > 1, then X may be the same or different;
**k** is an integer greater than or equal to 0;
**Y** is Cl or Br; wherein if k > 1, then Y may be the same or different;
**m** is an integer greater than or equal to 0;
each **Q** is independently F, Cl, Br, R¹, acyl, C(O)R¹, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R¹, N(R¹)SO₂R¹, N(acyl)₂, N[C(O)R¹]SO₂R¹, N[C(O)R¹]₂, CF₃; or any two vicinal Q substituents are joined to form a 5- or 6-membered substituted or unsubstituted, saturated or unsaturated carbocyclic or heterocyclic ring;
wherein each R¹ is independently aryl, alkyl, cycloalkyl or heterocyclyl, wherein said R¹ is optionally substituted by one or more substituents of R²;
wherein each R² is independently F, Cl, Br, COOH, acyl, aryl, alkyl, cycloalkyl or heterocyclyl; wherein if either one of R² in **(2A)** is carboxylic group COOH, then the respective R² in (**1A**) is Br or Cl;
wherein the position of said X, Y and Q in said structure of formula (**1A**) correspond to the same position of said COOH, Br and Q, respectively in said structure of formula **(2A).**

In one embodiment, this invention is directed to a process for the preparation of haloarene of formula (**1B**) 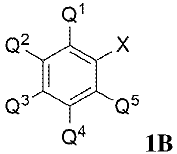 from a arenecarboxylic acid of formula **(2B),** 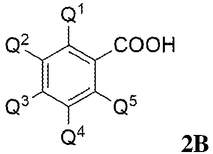 wherein said process comprises radical halo-de-carboxylation reaction comprising reacting **(2B)** with a chloroisocyanurate and a brominating agent to yield haloarene (**1B**); wherein,
said chloroisocyanurate is trichloroisocyanuric acid, dichloroisocyanuric acid, or any combination thereof
wherein
X is Cl or Br;
wherein Q¹, Q², Q³, Q⁴, and Q⁵, are each independently selected from: H, F, Cl, Br, R¹, COOH, acyl, C(O)R¹, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R², N(R¹)SO₂R¹, N(acyl)₂, N[C(O)R¹]SO₂R¹, N[C(O)R¹]₂, CF₃; or any two of Q¹ and Q², Q² and Q³, Q³ and Q⁴ , or Q⁴ and Q⁵, are joined to form a 5- or 6-membered substituted or unsubstituted, saturated or unsaturated carbocyclic or heterocyclic ring;
   wherein each R¹ is independently aryl, alkyl, cycloalkyl or heterocyclyl wherein R¹ is optionally substituted by one or more substituents of R²;
   wherein each R² is independently F, Cl, Br, COOH, acyl, aryl, alkyl, cycloalkyl or heterocyclyl;
   wherein if either one of Q¹, Q², Q³, Q⁴, Q⁵, and/or R² in **(2B)** is carboxylic group COOH, then the respective Q¹, Q², Q³, Q⁴, Q⁵, and/or R² in (**1B**) is Cl or Br;
   wherein if either one of Q¹, Q², Q³, Q⁴, Q⁵, and/or R² in **(2B)** is bromine (Br) then the respective Q¹, Q², Q³, Q⁴, Q⁵, and/or R² in (**1B**) is Cl or Br.

In one embodiment, this invention is directed to a radiation-sensitive composition comprising a carboxylic acid **(2A)** 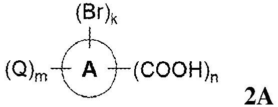 a chloroisocyanurate and a brominating agent which generates organic halide (**1A**) 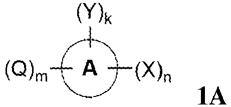 upon electromagnetic irradiation,
wherein
said chloroisocyanurate is trichloroisocyanuric acid, dichloroisocyanuric acid, or any combination thereof;
**A** is arene, branched alkane, cycloalkane or saturated heterocycle;
**n** is an integer greater than or equal to 1;
**m** is an integer greater than or equal to 0;
**X** is Cl or Br; wherein if n > 1, then X may be the same or different;
**k** is an integer greater than or equal to 0;
**Y** is Cl or Br; wherein if k > 1, then Y may be the same or different;
each **Q** is independently F, Cl, Br, R¹, acyl, C(O)R¹, C(O)OR¹, C(O)OMe, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R¹, N(R¹)SO₂R¹, N(acyl)₂, N[C(O)R¹]SO₂R¹, N[C(O)R¹]₂, CF₃; or any two vicinal Q substituents are joined to form a 5- or 6-membered substituted or unsubstituted, saturated or unsaturated carbocyclic or heterocyclic ring;
   wherein each R¹ is independently aryl, alkyl, cycloalkyl or heterocyclyl, wherein R¹ is optionally substituted by one or more substituents of R² ;
   wherein each R² is independently F, Cl, Br, COOH, acyl, aryl, alkyl, cycloalkyl or heterocyclyl;
   wherein if either one of R² in **(2A)** is a carboxylic group COOH, then the respective R² in (**1A**) is Br or Cl;
   wherein the position of said Br and Q in said structure of formula (**1A**) correspond to the same position of said COOH and Q, respectively in said structure of formula **(2A).**

In one embodiment, the process and composition of this invention comprises a brominating agent. In another embodiment, the brominating agent is Br₂ (bromine), a salt comprising bromide or a polybromide anion and an organic or inorganic cation; or any combination thereof.

In one embodiment, the process of this invention is conducted in the presence of an organic or inorganic solvent and the composition of this invention comprises an organic or inorganic solvent. In another embodiment, the inorganic solvent is CO₂, SO₂, SO₂Cl₂ or combination thereof. In another embodiment, the solvent is CH₃CN, CH₃NO₂, an ester, a hydrocarbon solvent, or halocarbon solvent or combination thereof. In another embodiment, the hydrocarbon solvent is C₆H₆. In another embodiment, the halocarbon solvent is CH₂Cl₂, Cl(CH₂)₂Cl, CHCl₃, CCl₄, C₆H₅Cl, *o*-C₆H₄Cl₂, BrCCl₃, CH₂Br₂, CFCl₃, CF₃CCl₃, ClCF₂CFCl₂, BrCF₂CFClBr, CF₃CClBr₂, CF₃CHBrCl, C₆H₅F, C₆HₛCF₃, 4-ClC₆H₄CF₃, 2,4-Cl₂C₆H₃CF₃ or any combination thereof.

In one embodiment, the process and composition of this invention comprises and is further subjected to electromagnetic irradiation. In another embodiment, the electromagnetic irradiation is microwave, infrared, ultraviolet, or visible light irradiation or any combination thereof. In another embodiment, the electromagnetic irradiation is visible light irradiation. In another embodiment, the source of said visible light is sunlight, fluorescent lamp, light-emitting diode, incandescent lamp or any combination thereof.

In one embodiment, the process and composition of this invention comprises a brominating agent and a carboxylic acid compound of formula **(2A)** or **(2B).** In another embodiment, the molar ratio between brominating agent / (each carboxylic group of said carboxylic acid compound) is between 0.1 and 4.

In one embodiment, the process and composition of this invention comprises chloroisocyanurate and a carboxylic acid compound of formula **(2A)** or **(2B).** In another embodiment, the molar ratio between the chloroisocyanurate / (each carboxylic group of the carboxylic acid compound) is between 0.1 and 2.

In another embodiment, the process of this invention is conducted at a temperature of between about -20 °C and about 200 °C. In another embodiment, the process of this invention is conducted at a temperature of between about 0 °C and about 150 °C.

In another embodiment, the process is conducted in the presence of radical initiator. In another embodiment, the radical initiator is an azo compound or organic peroxide.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

In one embodiment, this invention is directed to a process for the preparation of organic halide of formula (**1A**) from a carboxylic acid of formula **(2A)** represented by scheme 1: 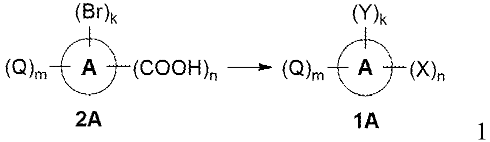 said process comprises radical halo-de-carboxylation reaction comprising reacting carboxylic acid **(2A)** with a chloroisocyanurate and a brominating agent to yield organic halide (**1A**);
wherein
said chloroisocyanurate is trichloroisocyanuric acid, dichloroisocyanuric acid, or any combination thereof;
**A** is arene, alkane, cycloalkane or saturated heterocycle;
**n** is an integer greater than or equal to 1;
**m** is an integer greater than or equal to 0;
**X** is Cl or Br; wherein if n > 1, then X may be the same or different;
**k** is an integer greater than or equal to 0;
**Y** is Cl or Br; wherein if k > 1, then Y may be the same or different;
each **Q** is independently F, Cl, Br, R¹, acyl, C(O)R¹, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R¹, N(R¹)SO₂R¹, N(acyl)₂, N[C(O)R¹]SO₂R¹, N[C(O)R¹]₂, CF₃; or any two vicinal Q substituents are joined to form a 5- or 6-membered substituted or unsubstituted, saturated or unsaturated carbocyclic or heterocyclic ring;
wherein each R¹ is independently aryl, alkyl, cycloalkyl or heterocyclyl, wherein said R¹ is optionally substituted by one or more substituents of R²;
wherein each R² is independently F, Cl, Br, COOH, acyl, aryl, alkyl, cycloalkyl or heterocyclyl; wherein if either one of R² in **(2A)** is carboxylic group COOH, then the respective R² in (**1A**) is Br or Cl;
wherein the position of said X, Y and Q in said structure of formula (**1A**) correspond to the same position of said COOH, Br and Q, respectively in said structure of formula **(2A).**

In one embodiment, this invention is directed to a process for the preparation of organic halide of formula (**1B**) from a carboxylic acid of formula **(2B)** represented by scheme 2: 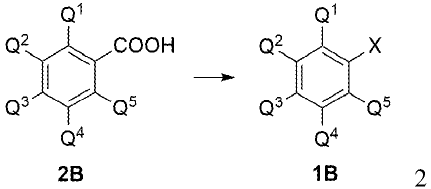 wherein said process comprises radical halo-de-carboxylation reaction comprising reacting **(2B)** with a chloroisocyanurate and a brominating agent to yield haloarene (**1B**); wherein,
said chloroisocyanurate is trichloroisocyanuric acid, dichloroisocyanuric acid, or any combination thereof
wherein
X is Cl or Br;
wherein Q¹, Q², Q³, Q⁴, and Q⁵, are each independently selected from: H, F, Cl, Br, R¹, COOH, acyl, C(O)R¹, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R², N(R¹)SO₂R¹, N(acyl)₂, N[C(O)R¹]SO₂R¹, N[C(O)R¹]₂, CF₃; or any two of Q¹ and Q², Q² and Q³, Q³ and Q⁴ , or Q⁴ and Q⁵, are joined to form a 5- or 6-membered substituted or unsubstituted, saturated or unsaturated carbocyclic or heterocyclic ring;
   wherein each R¹ is independently aryl, alkyl, cycloalkyl or heterocyclyl wherein R¹ is optionally substituted by one or more substituents of R²;
   wherein each R² is independently F, Cl, Br, COOH, acyl, aryl, alkyl, cycloalkyl or heterocyclyl;
   wherein if either one of Q¹, Q², Q³, Q⁴, Q⁵, and/or R² in **(2B)** is carboxylic group COOH, then the respective Q¹, Q², Q³, Q⁴, Q⁵, and/or R² in (**1B**) is Cl or Br;
   wherein if either one of Q¹, Q², Q³, Q⁴, Q⁵, and/or R² in **(2B)** is bromine (Br) then the respective Q¹, Q², Q³, Q⁴, Q⁵, and/or R² in (**1B**) is Cl or Br.

In one embodiment, the organic halide, (**1A**) or (**1B**) that is formed in the process of this invention is a mixture of the respective chloride and bromide of each compound. In another embodiment, the organic halide of formula (**1A**) is a mixture of organic halide products of formula (**1A**) with different X groups; if k is different than 0, then the organic halide products optionally have also different Y group. In another embodiment, the organic halide of formula (**1B**) is a mixture of organic halide products of formula (**1B**) with different X groups; if Q¹, Q², Q³, Q⁴, Q⁵ is Cl or Br , then the organic halide products optionally are a mixture of different halo groups (Cl or Br).

In one embodiment, **A** of the organic halide (**1A**) and of the carboxylic acid **(2A)** in scheme 1 is arene. In another embodiment, **A** of the organic halide (**1A**) and the carboxylic acid **(2A)** in scheme 1 is a alkane. In another embodiment, **A** of the organic halide (**1A**) and the carboxylic acid **(2A)** in scheme 1 is a linear alkane. In another embodiment, **A** of the organic halide (**1A**) and the carboxylic acid **(2A)** in scheme 1 is a branched alkane. In another embodiment, **A** of the organic halide (**1A**) and of the carboxylic acid **(2A)** in scheme 1 is a cycloalkane. In another embodiment, **A** of the organic halide (**1A**) and of the carboxylic acid **(2A)** in scheme 1 is a saturated heterocycle.

In one embodiment the **A** is substituted with one or more substituents **Q** (in Scheme 1); where each **Q** is independently F, Cl, Br, R¹, acyl, C(O)R¹, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R¹, N(R¹)SO₂R¹, N(acyl)₂, N[C(O)R¹]SO₂R¹, N[C(O)R¹]₂, CF₃; or any two vicinal Q substituents are joined to form a 5- or 6-membered substituted or unsubstituted, saturated or unsaturated carbocyclic or heterocyclic ring;
wherein each **R¹** is independently aryl, alkyl, cycloalkyl or heterocyclyl, wherein R¹ is optionally substituted by one or more substituents of R² ;
wherein each **R²** is independently F, Cl, Br, COOH, acyl, aryl, alkyl, cycloalkyl or heterocyclyl.

In another embodiment, each **Q** is independently Cl. In another embodiment, each **Q** is independently F. In another embodiment, each **Q** is independently Br. In another embodiment, each **Q** is independently CN. In another embodiment, each **Q** is independently CF₃. In another embodiment, each **Q** is independently CCl₃. In another embodiment, each **Q** is independently acyl group. In another embodiment, each **Q** is independently SO₃R¹. In another embodiment, each **Q** is independently SO₂R¹. In another embodiment, each **Q** is independently C(O)R¹. In another embodiment, each **Q** is independently C(O)OR¹. In another embodiment, each **Q** is independently C(O)OMe. In another embodiment, each **Q** is independently COCl. In another embodiment, each **Q** is independently amide. In another embodiment, each **Q** is independently C(O)N(R¹)₂. In another embodiment, each **Q** is independently OCF₃. In another embodiment, each **Q** is independently R¹. In another embodiment, each **Q** is independently alkyl. In another embodiment, each **Q** is independently *t*-Bu. In another embodiment, each **Q** is independently cycloalkyl. In another embodiment, each **Q** is independently heterocyclyl. In another embodiment, each **Q** is independently OR¹. In another embodiment, each **Q** is independently OMe. In another embodiment, each **Q** is independently SR¹. In another embodiment, each **Q** is independently SMe. In another embodiment, each **Q** is independently acetyl. In another embodiment, each **Q** is independently benzoyl. In another embodiment, each **Q** is independently mesyl. In another embodiment, each **Q** is independently tosyl. In another embodiment, each **Q** is independently NO₂. In another embodiment, each **Q** is independently N(R¹)₃⁺. In another embodiment, each **Q** is independently O-acyl. In another embodiment, each **Q** is independently OC(O)R¹. In another embodiment, each **Q** is independently acetoxy. In another embodiment, each **Q** is independently OSO₂R¹. In another embodiment, each **Q** is independently mesyloxy. In another embodiment, each **Q** is independently tosyloxy. In another embodiment, each **Q** is independently S-acyl. In another embodiment, each **Q** is independently SC(O)R¹. In another embodiment, each **Q** is independently N(R¹)acyl. In another embodiment, each **Q** is independently N(R¹)C(O)R¹. In another embodiment, each **Q** is independently N(R¹)SO₂R¹. In another embodiment, each **Q** is independently N(acyl)₂. In another embodiment, each **Q** is independently N[C(O)R¹]SO₂R¹. In another embodiment, each **Q** is independently saccharinyl. In another embodiment, each **Q** is independently N[C(O)R¹]₂. In another embodiment, each **Q** is independently phthalimido. In another embodiment, each **Q** is independently aryl. In another embodiment, each **Q** is independently C₆H₅. In another embodiment, each **Q** is independently C₆F₅. In another embodiment, two vicinal **Q** substituents are joined to form a 5- or 6-membered substituted or unsubstituted, saturated or unsaturated heterocyclic ring. In another embodiment, two vicinal **Q** substituents are joined to form dihydrofuran-2,5-dione. In another embodiment, two vicinal **Q** substituents are joined to form pyrrolidine-2,5-dione. In another embodiment, if m> 1 then **Q** substituents are the same. In another embodiment, if m>1 then **Q** substituents are different.

In one embodiment, **A** of the organic bromide (**1A**) and of the carboxylic acid **(2A)** in scheme 1 is a benzene. In another embodiment, **A** is cycloalkane. In another embodiment, **A** is a saturated heterocycle.

In another embodiment **A** of the organic bromide (**1A**) and of the carboxylic acid **(2A)** in scheme 1 is an alkane. In another embodiment, the alkane chain is linear. In another embodiment, the alkane chain is branched.

In one embodiment, the carboxylic acid **(2A)** in scheme 1 and the carboxylic acid **(2B)** in scheme 2 is not ECH(Z)-COOH, wherein E is acyl, CO₂Z', SO₂Z', S(Z')₂⁺, or N(Z')₃⁺ and Z and Z' are each independently a hydrogen, alkyl or an aryl. In another embodiment, the carboxylic acid **(2A)** in scheme 1 and the carboxylic acid **(2B)** in scheme 2 is not ZCH=CH-COOH or ZC=C-COOH, where Z is either a hydrogen, alkyl or an aryl, the latter two are optionally substituted.

In another embodiment, the **A** in scheme 1 is not unsaturated heterocycle. In another embodiment, the **A** in scheme 1 is not alkene or alkyne. In another embodiment, the **A** in scheme 1 is not cycloalkene or cycloalkyne. In another embodiment, the **Q** in scheme 1 is not OH, NH₂, NHR, or NR² group.

In another embodiment, at least one of **Q¹**, **Q²**, **Q³**, **Q⁴**, and/or **Q⁵** is F, Cl, Br, CF₃, CCl₃, CN, COOH, C(O)OMe, NO₂, phthalimide, OCF₃, and/or any two of **Q¹** and **Q²**, **Q²** and **Q³**, **Q³** and **Q⁴**, or **Q⁴** and **Q⁵**, are joined to form a dihydrofuran-2,5-dione or pyrrolidine-2,5-dione ring.

In another embodiment, at least one of **Q¹**, **Q²**, **Q³**, **Q⁴**, and **Q⁵** is NO₂. In another embodiment, at least one of **Q¹**, **Q²**, **Q³**, **Q⁴**, and **Q⁵** is CF₃. In another embodiment, at least one of **Q¹**, **Q²**, **Q³**, **Q⁴**, and **Q⁵** is CN. In another embodiment, at least one of **Q¹**, **Q²**, **Q³**, **Q⁴**, and **Q⁵** is Cl. In another embodiment, at least one of **Q¹**, **Q²**, **Q³**, **Q⁴**, and **Q⁵** is F. In another embodiment, at least one of **Q¹**, **Q²**, **Q³**, **Q⁴**, and **Q⁵** is Br. In another embodiment, at least one of **Q¹**, **Q²**, **Q³**, **Q⁴**, and **Q⁵** is phthalimide. In another embodiment, at least one of **Q¹**, **Q²**, **Q³**, **Q⁴**, and **Q⁵** is C(O)OMe.

In one embodiment, **Q¹** of formula (**1B**) and **(2B)** in scheme 2 is F. In another embodiment, **Q¹** is H. In another embodiment, **Q¹** is CF₃. In another embodiment, **Q₁** is Cl. In another embodiment, **Q¹** is Br. In another embodiment, **Q¹** is NO₂. In another embodiment, **Q¹** is CO₂Me. In another embodiment, **Q¹** is phthalimide.

In one embodiment, **Q²** of formula (**1B**) and **(2B)** in scheme 2 is H. In another embodiment, **Q²** is F. In another embodiment, **Q²** is CF₃. In another embodiment, **Q²** is Cl. In another embodiment, **Q²** is Br. In another embodiment, **Q²** is CN. In another embodiment, **Q²** is NO₂. In another embodiment, **Q²** is CO₂Me. In another embodiment, **Q²** is COOH.

In one embodiment, **Q³** of formula (**1B**) and **(2B)** in scheme 2 is H. In another embodiment, **Q³** is CN. In another embodiment, **Q³** is Cl. In another embodiment, **Q³** is Br. In another embodiment, **Q³** is F. In another embodiment, **Q³** is CF₃. In another embodiment, **Q³** is NO₂. In another embodiment, **Q³** is CO₂Me. In another embodiment, **Q³** is COOH.

In one embodiment, **Q⁴** of formula (**1B**) and **(2B)** in scheme 2 is H. In another embodiment, **Q⁴** is F. In another embodiment, **Q⁴** is CF₃. In another embodiment, **Q⁴** is CN. In another embodiment, **Q⁴** is Cl. In another embodiment, **Q⁴** is NO₂.

In one embodiment, **Q⁵** of formula (**1B**) and **(2B)** in scheme 2 is H. In another embodiment, **Q⁵** is F. In another embodiment, **Q⁵** is CF₃. In another embodiment, **Q⁵** is CN. In another embodiment, **Q⁵** is Cl.

In one embodiment, **Q³** and **Q⁴** of formula (**1B**) and **(2B)** in scheme 2 are joined to form a 5- or 6-membered substituted or unsubstituted, saturated or unsaturated heterocyclic ring. In another embodiment, the heterocyclic ring is dihydrofuran-2,5-dione. In another embodiment, the heterocyclic ring is pyrrolidine-2,5-dione. In another embodiment, the heterocyclic ring is substituted with an alkyl. In another embodiment, the alkyl is *t*-Bu.

In one embodiment, **X** of scheme 1 and of compounds (**1A**) and (**1B**) is Br or Cl. In another embodiment **X** is Cl. In another embodiment **X** is Br.

In one embodiment, **Y** of scheme 1 and of compounds (**1A**) is Br or Cl. In another embodiment **Y** is Cl. In another embodiment **Y** is Br.

In another embodiment, the compound of formula (**1A**) or (**1B**) comprises a both Cl and Br. In another embodiment, the process Scheme 1 or Scheme 2 yields a mixture of products of formula (**1A**) or (**1B**) wherein each product has different halo groups.

In one embodiment, **m** of scheme 1 and of compounds (**1A**) and **(2A)** is an integer number greater than or equal to 0. In another embodiment, **m** is 0. In another embodiment, **m** is 1. In another embodiment, **m** is 2. In another embodiment, **m** is 3. In another embodiment, if m>1 than Q can be different or the same.

In one embodiment, **n** of compounds (**1A**), **(2A)** in scheme 1 is an integer number greater than or equal to 1. In another embodiment, **n** is between 1 and 5. In another embodiment, **n** is between 1 and 3. In another embodiment, **n** is 1 or 2. In another embodiment, **n** is 1. In another embodiment, **n** is 2. In another embodiment, **n** is 3. In one embodiment if n > 1, then X may be the same or different.

In one embodiment, **k** of scheme 1 and of compounds (**1A**) and **(2A)** is an integer number greater than or equal to 0. In another embodiment, **k** is 0. In another embodiment, **k** is 1. In another embodiment, **k** is 2. In another embodiment, **k** is 3. In another embodiment, if k>1 than Y may be the same or different.

In one embodiment, this invention is directed to a process for the preparation of organic halides from its corresponding carboxylic acid, said process comprises a radical halo-de-carboxylation reaction of the carboxylic acid with a chloroisocyanurate and a brominating agent, wherein said carboxylic acid is selected from the carboxylic acids listed in Tables 2, 3, 4 and 7 below.

In one embodiment, X and Y of the organic halide (**1A**) are bromine atoms when **A** is an alkane, cycloalkane or saturated heterocycle.

In one embodiment, X of the organic halide (**1A**) is a bromine atom if **Q** is a nitro group. In one embodiment, X of the organic halide (**1B**) is a bromine atom if either one of Q¹, Q², Q³, Q⁴, Q⁵ is a nitro group.

In one embodiment, X of the organic halide (**1A**) is a chlorine atom if **Q** is different than a nitro group. In one embodiment, X of the organic halide (**1B**) is a chlorine atom if none of the substituents Q¹, Q², Q³, Q⁴ and Q⁵ are a nitro group.

In one embodiment, if either one of Q¹, Q², Q³, Q⁴, or Q⁵ in arenecarboxylic acid **(2B)** is Br then the respective Q¹, Q², Q³, Q⁴, or Q⁵ in haloarene (**1B**) is Cl.

In one embodiment, the process of this invention, represented by schemes 1 and 2, has a radical mechanism. In another embodiment all factors that promote radical reaction may stimulate the process of this invention. Factors that promote radical reaction: heating, electromagnetic irradiation, addition of radical initiators.

In one embodiment, the process and composition of this invention comprises chloroisocyanurate. In another embodiment, the chloroisocyanurate is trichloroisocyanuric acid (TCCA) dichloroisocyanuric acid (DCCA), salts thereof or any combination thereof.

In one embodiment, the process of this invention and the composition of this invention make use and comprises a brominating agent. In another embodiment, the brominating agent is bromine, a salt comprising bromide or a polybromide anion and an organic or inorganic cation; or any combination thereof.

In another embodiment, the cation is a substituted or unsubstituted onium ion. The term "onium" refers in one embodiment to cations (with their counter-ions) derived by addition of a hydron to a mononuclear parent hydride of the nitrogen, chalcogen and halogen families. Non limiting examples of oniums include [NH₄]⁺ ammonium, [OH₃]⁺ oxonium, [PH₄]⁺ phosphonium, [SH₃]⁺ sulfonium, [AsH₄]⁺ arsonium, [SeH₃]⁺ selenonium, [BrH₂]⁺ bromonium, [SbH₄]⁺ stibonium, [TeH₃]⁺) telluronium, [IH₂]⁺ iodonium, [BiH₄]⁺ bismuthonium.

Substituted oniums refers to substitution of the above parent ions by univalent groups or by two or three free valencies. E.g. [SMe₃]⁺ trimethylsulfonium (a tertiary sulfonium ion), [MePPh₃]⁺ methyltriphethylphosphonium (a quaternary phosphonium ion), [HNEt₃]⁺ triethylammonium (a tertiary ammonium ion), [NPr₄]⁺ tetrapropylammonium (a quaternary ammonium ion), [R₂C=NR₂]⁺ iminium ions.

In one embodiment, the term "inorganic cation" used herein refers to an alkali or alkaline earth metal cations, transition metal cation, or unsubstituted onium cation. In another embodiment, the inorganic cation is Li⁺. In another embodiment, the inorganic cation is Na⁺. In another embodiment, the inorganic cation is K⁺. In another embodiment, the inorganic cation is Rb⁺. In another embodiment, the inorganic cation is Cs⁺. In another embodiment, the inorganic cation is Zn²⁺. In another embodiment, the inorganic cation is Cu²⁺. In another embodiment, the inorganic cation is ammonium cation [NH₄]⁺.

In one embodiment, the term "organic cation" used herein refers to substituted onium cation. In another embodiment, the substituted onium cation is substituted ammonium cation, substituted phosphonium cation, substituted oxonium cation, substituted sulfonium cation, substituted arsonium cation, substituted selenonium cation, substituted telluronium cation, substituted iodonium cation, any other known onium cation, or any combination thereof. In another embodiment, the substituted ammonium cation is the substituted or unsubstituted guanidinium cation, substituted or unsubstituted pyridinium cation, substituted or unsubstituted amidinium cation, substituted or unsubstituted quaternary ammonium cation [NR¹₄]⁺, substituted or unsubstituted tertiary ammonium cation [HNR¹₃]⁺. In another embodiment, the substituted phosphonium cation is substituted or unsubstituted quaternary phosphonium cation [PR¹₄]⁺; wherein R¹ is alkyl, aryl, cycloalkyl, heterocyclyl, or any combination thereof. In another embodiment, the quaternary ammonium cation [NRS¹₄]⁺ is tetraalkylammonium, trialkylarylammonium, dialkyldiarylammonium, trialkylbenzylammonium, or any combination thereof. In another embodiment, non-limiting examples of the quaternary ammonium cation [NR¹₄]⁺ include tetrametylammonium, tetraethylammonium, tetrabutylammonium, tetraoctylammonium, trimethyloctylammonium, cetyltrimethylammonium, or any combination thereof. In another embodiment, the quaternary phosphonium cation [PR¹₄]⁺ is tetraalkylphosphonium, alkyltriarylphosphonium, benzyltriarylphosphonium, benzyltrialkylphosphonium, or any combination thereof. In another embodiment, non-limiting examples of the quaternary phosphonium cation [PR¹4]⁺ include tetraphenylphosphonium, benzyltriphenylphosphonium, tetrabutylphosphonium, methyltriphenylphosphonium, benzyltributylphosphonium cation or any combination thereof. In another embodiment, the substituted sulfonium cation is substituted or unsubstituted tertiary sulfonium cation, substituted or unsubstituted sulfoxonium, thiopyrylium or thiuronium ion; or any combination thereof. In another embodiment the substituted oxonium cation is substituted or unsubstituted tertiary oxonium cation, substituted or unsubstituted pyrylium cation; or any combination thereof. In another embodiment, substituted cations as referred herein are substituted with halide, nitrile, nitro, alkyl, aryl, cycloalkyl, heterocyclyl, amide, carboxylic acid, acyl or any combination thereof.

In one embodiment, the term "polybromide anion" used herein refers to a molecule or ion containing three or more bromine atoms or to an ion of formula [Brₚ]^{q-}, where **p** is an integer of at least 3 and **q** is an integer of at least 1 and not more than p/2. In another embodiment, **p** is an integer between 3-24 and **q** is 1 or 2. In another embodiment **p** is 3, 5, 7, 9, 11 or 13 and **q** is 1. In another embodiment **p** is 4, 8, 20 or 24 and **q** is 2.

In another embodiment, the brominating agent is Br₂, Bu₄NBr, Bu₄NBr₃, or any combination thereof.

An "alkyl" refers, in one embodiment, to a univalent groups derived from alkanes by removal of a hydrogen atom from any carbon atom: CₙH₂ₙ₊₁-. In one embodiment, the alkyl group has 1-20 carbons. Examples for alkyls include but are not limited to: methyl, ethyl, propyl, isopropyl, butyl, isobutyl, *s*-butyl, *tert*-butyl*,* pentyl, neopentyl, octyl, isooctyl and the like

The term "alkane" refers to acyclic branched or unbranched hydrocarbons having the general formula C*ₙ*H_{2*n*+2}, and therefore consisting entirely of hydrogen atoms and saturated carbon atoms. Examples of alkane include: methane, ethane, propane, *n-*butane, isobutane, *n*-pentane, neopentane, *n*-octane, isooctane and the like.

An "arene" refers to monocyclic and polycyclic aromatic hydrocarbons. Nonlimiting examples of arenes are benzene, biphenyl, naphthalene, anthracene, and the like.

An "aryl" group refers, to univalent groups derived from arenes by removal of a hydrogen atom from a ring carbon atom. Nonlimiting examples of aryl groups are phenyl, naphthyl, antracenyl, phenanthryl, and the like.

A "cycloalkyl" refers to univalent groups derived from cycloalkanes by removal of a hydrogen atom from a ring carbon atom Non limiting examples of cycloalkyl include: cyclobutyl, norbornyl, cyclopentyl and cyclohexyl.

A "cycloalkane" refers to saturated mono- or polycyclic hydrocarbons. A general chemical formula for cycloalkanes would be CₙH_{2(n+1-g)} where n = number of C atoms and g = number of rings in the molecule.

A "heterocyclyl" refers to univalent groups formed by removing a hydrogen atom from any ring atom of a mono or polycyclic heterocyclic compound.

A "heterocycle" refers to a mono- or poly-cyclic heterocyclic compound consisting of carbon, hydrogen and at least one of nitrogen, sulfur, oxygen, phosphorous or combination thereof in one of the rings. In one embodiment, the heterocyclic compound consists 2-7 fused rings. Non limiting examples of monocyclic saturated heterocyclic compounds are aziridine, oxirane, thiirane, azetidine, oxetane, thietane, pyrrolidine, tetrahydrofurane, thiolane, pyperidine, oxane, thiane, azepane, oxepane, thiepane, imidazolidine, oxazolidine, thiazolidine, dioxolane, piperazine, morpholine, dioxane, homopiperazine. Non limiting examples of saturated bicyclic heterocyclic compounds are quinuclidine, 7-oxanorbornane, 7-thiabicyclo[2.2.1]heptane, 3-oxabicyclo[3.1.1]heptane, 3-azabicyclo[3.1.1]heptane, octahydroindole, octahydro-2-benzofuran.

An "amide" refers, in one embodiment, to a derivative of oxoacid in which an acidic hydroxyl group has been replaced by an amino or substituted amino group. Compounds having one or two acyl groups on a given nitrogen are generically included and may be designated as primary and secondary amides, respectively.

An "acyl" group is formed by removing one or more hydroxyl groups from oxoacids, and replacement analogues of such acyl groups. E.g. -C(=O)R, -C(=O)OR,-C(=O)NR₂, -C=N, -S(=O)₂R, -S(=O)₂OR, -NO₂. Non limiting examples of the acyl groups include acetyl -C(O)Me, benzoyl -C(O)Ph, C(O)OMe, -C(=O)Cl, mesyl MeSO₂-, tosyl 4-MeC₆H₄SO₂.

A "carboxylic acid" refers, in one embodiment, to oxoacids having the structure RC(=O)OH.

In another embodiment, the halo-de-carboxylation reaction represented by schemes 1 and 2 is conducted at room temperature. In another embodiment, the reaction is conducted under cooling. In another embodiment, the halo-de-carboxylation reaction is initiated thermally. In another embodiment, the halo-de-carboxylation reaction is further subjected to heat. In another embodiment, the halo-de-carboxylation reaction is conducted at a temperature of between -20 °C and 200 °C. In another embodiment, said process is conducted at a temperature of between about 0 °C and about 150 °C.

In another embodiment, the process of this invention further comprising the use of radical initiator in the reaction. In another embodiment, the radical initiator is an azo compound or organic peroxide. In another embodiment, the azo compound is azobisisobutyronitrile (AIBN) or 1,1'-azobis(cyclohexanecarbonitrile) (ABCN). In another embodiment, the organic peroxide is benzoyl peroxide.

In another embodiment, the bromoarene of formula (**1A**) and/or (**1B**) is prepared according to process described in Examples 1-14.

In one embodiment, the process of this invention, represented by schemes 1 and 2, is conducted under electromagnetic irradiation. In another embodiment, the electromagnetic radiation is visible light, infrared radiation, ultraviolet radiation, microwave radiation or any combination thereof.

In another embodiment, the source of the visible light is sunlight, fluorescent lamp, light-emitting diode, incandescent lamp or any combination thereof.

The term "irradiation" refers in one embodiment to the energy that is irradiated or transmitted in the form of rays or waves or particles. Electromagnetic irradiation refers to radiation consisting of waves of energy associated with electric and magnetic fields resulting from the acceleration of an electric charge. Ultrasound refers to cyclic mechanical vibrations with a frequency greater than 20 kilohertz (20,000 hertz). Ultraviolet irradiation refers to electromagnetic radiation with wavelengths 100 to 400 nm. Visible irradiation (light, visible light) refers to electromagnetic irradiation with wavelengths 400 to 780 nm. Infrared irradiation refers to electromagnetic irradiation with wavelengths 780 to 20000 nm. Microwave irradiation refers to electromagnetic irradiation with wavelengths 2 to 1000 mm.

Devices serving as a source of the electromagnetic irradiation include a mercury lamp, a xenon lamp, a carbon arc lamp, an incandescent lamp, a tungsten lamp, a fluorescent lamp, light-emitting diode, and sunlight, and the like.

Tungsten lamp refers to incandescent lamp that generates light by passing an electric current through a thin filament wire (usually of wolfram) until it is extremely hot. The lamps are often filled by a halogen gas such as iodine and bromine that allow filaments to work at higher temperatures and higher efficiencies.

Light-emitting diode (LED) refers to a semiconductor (often a combination of gallium, arsenic, and phosphorous or gallium and nitrogen) containing an n region (where electrons are more numerous than positive charges) separated from a p region (where positive charges are more numerous than negative charges). Upon application of a voltage, charges move and emission of ultraviolet, visible, or infrared radiation is produced each time a charge recombination takes place. Although an LED emits incoherent monochromatic light, normally a very narrow frequency range is obtained.

In another embodiment, the process is conducted in the presence of an organic or inorganic solvent and the composition of this invention comprises an organic or inorganic solvent. In another embodiment, the inorganic solvent is CO₂, SO₂, SO₂Cl₂ or combination thereof. In another embodiment, the organic solvent is CH₃CN, CH₃NO₂, an ester, a hydrocarbon solvent, or halocarbon solvent or combination thereof. In another embodiment the halocarbon solvent is CH₂Cl₂, Cl(CH₂)₂Cl, CHCl₃, CCl₄, C₆HₛCl, *o*-C₆H₄Cl₂, BrCCl₃, CH₂Br₂, CFCl₃, CF₃CCl₃, ClCF₂CFCl₂, BrCF₂CFClBr, CF₃CClBr₂, CF₃CHBrCl, C₆H₅F, C₆H₅CF₃, 4-ClC₆H₄CF₃, 2,4-Cl₂C₆H₃CF₃ or any combination thereof. In another embodiment, the solvent is CH₂Cl₂. In another embodiment, the solvent is a polar solvent. In another embodiment, the solvent is a nonpolar solvent. In another embodiment, the solvent is a hydrocarbon. In another embodiment, the solvent is benzene C6H6 (PhH). In another embodiment, the solvent is acetonitrile CH₃CN (MeCN). In another embodiment, the solvent is ethyl acetate EtOAc. In another embodiment, the solvent is halocarbon. In another embodiment, the solvent is CCl₄. In another embodiment, the solvent is chloroform CHCl₃. In another embodiment, the solvent is BrCCl₃. In another embodiment, the solvent is CH₂Br₂. In another embodiment, the solvent is CFCl₃. In another embodiment, the solvent is CF₃CCl_{3.} In another embodiment, the solvent is ClCF₂CFCl₂. In another embodiment, the solvent is BrCF₂CFClBr. In another embodiment, the solvent is CF₃CClBr₂. In another embodiment, the solvent is halothane CF₃CHBrCl. In another embodiment, the solvent is C₆H₅F. In another embodiment, the solvent is chlorobenzene C₆H₅Cl (PhCl). In another embodiment, the solvent is benzotrifluoride C₆H₅CF₃ (PhCF₃). In another embodiment, the solvent is 4-ClC₆H₄CF₃. In another embodiment, the solvent is 1,2-dichloroethane Cl(CH₂)₂Cl (DCE). In another embodiment, the solvent is *ortho*-dichlorobenzene *o-*C₆H₄Cl₂. In another embodiment, the solvent is dichloromethane CH₂Cl₂ (DCM). In another embodiment, the solvent is 2,4-dichlorobenzotrifluoride 2,4-Cl₂C₆H₃CF₃. In another embodiment, bromodecarboxylation process is preferably conducted in a halocarbon solvent. In another embodiment, bromodecarboxylation process is preferably conducted in a BrCCl₃, CH₂Cl₂, CH₂Br₂, CF₃CHBrCl or any combination thereof.

The term "hydrocarbon solvent" refers to any solvent consisting of the carbon and hydrogen elements. Non limiting examples of hydrocarbon solvents are cyclohexane, heptane, pentane, hexane, or benzene C6H6.

The term "halocarbon solvent" refers to any solvent wherein one or more of the carbons are covalently linked to one or more halogens (fluorine, chlorine, or bromine). Non limiting examples of halocarbon solvents are chloroform CHCl₃, dichloromethane CH₂Cl₂ (DCM), bromotrichloromethane BrCCl₃, chlorobenzene C₆H₅Cl (PhCl), *ortho-*dichlorobenzene *o*-C₆H₄Cl₂, 1,2-dichloroethane Cl(CH₂)₂Cl (DCE), carbon tetrachloride CCl₄, 1,3-dichloropropane Cl(CH₂)₃Cl, 1,1,2,2-tertrachlorodifluoroethane FCCl₂CCl₂F, 1,1,2-trichloroethane CHCl₂CH₂Cl, trichloroethylene Cl₂C=CHCl, perchloroethylene Cl₂C=CCl₂, bromobenzene C₆H₅Br, 1,1,2-trichlorotrifluoroethane, dibromomethane CH₂Br₂, 2-bromo-2-chloro-1,1,1-trifluoroethane CF₃CHBrCl (halothane), 1,2-dibromoethane Br(CH₂)₂Br, benzotrifluoride C₆H₅CF₃ (PhCF₃), 2,4-dichlorobenzotrifluoride 2,4-Cl₂C₆H₃CF₃.

In one embodiment, the process of this invention, represented by schemes 1 and 2 is conducted in the presence of electromagnetic irradiation. In another embodiment, the shorter the time of the reaction, the larger the obtained amount of the bromide product with respect to the chloride. In another embodiment, the longer the time of the reaction, the larger the obtained amount of the chloride product with respect to the bromide. In one embodiment, when the process is conducted for a time less than 1 h, the major product is the bromide. In another embodiment, when the process is conducted for a time longer than 3 h, the major product is the chloride.

In one embodiment, following the formation of organic halide, or the compound of formula (**1A**) or (**1B**) the organic halide is isolated from the reaction mixture by filtration, washing, chromatography, crystallization or any combination thereof. In one embodiment, following the formation of R-X, R-X is isolated from the reaction mixture by filtration, washing, chromatography, crystallization or any combination thereof. In another embodiment the compound of formula (**1A**) or (**1B**) is isolated from the reaction mixture by filtration followed by a washing step. In another embodiment the washing step comprises washing with an aqueous reducing agent followed by washing with an aqueous base. In another embodiment the washing step comprises washing with an aqueous base followed by washing with an aqueous reducing agent. In another embodiment, the washing step comprises washing with an aqueous reducing agent and a base.

In one embodiment the bromide compound, chloride compound of formula (**1A**) or (**1B**) or mixture thereof is isolated from the reaction mixture by a washing step.

In another embodiment, the washing step comprises treating of the reaction mixture with reducing agent, wherein excess of the chloroisocyanurate is converted to cyanuric acid insoluble in non-polar organic solvents, and thereby can be removed from the organic phase. In another embodiment, an aqueous reducing agent refers to an aqueous solution comprising a reducing agent. Non limiting examples of reducing agents are Na₂SO₃, NaHSO₃, Na₂S₂O₃, NaBH₄/NaOH or combination thereof. In another embodiment the reducing agent is added at a concentration of between 1-10% w/w to the water to obtain an aqueous reducing agent solution.

In one embodiment, the process of this invention directed to halo-de-carboxylation comprising a washing step with an aqueous reducing agent. In another embodiment, following the washing step a potassium iodide starch paper test is performed to identify traces of the bromo reagent. "A potassium iodide starch paper test" (SPT) refers to a starch iodide test paper that has been wetted with aqueous acetic acid; 1:1; v/v]. In another embodiment, if the test is positive, an additional aqueous reducing agent is added to the reaction mixture.

In another embodiment the washing step comprises washing the product with a mild aqueous base wherein the unreacted carboxylic acid is removed from the organic phase by washing with an aqueous base. In another embodiment, the carboxylic acid is recovered by acidifying the aqueous phase. In another embodiment, an aqueous base refers to an aqueous solution comprising a base. Non limiting examples of a base is NaHCO₃, NaOH, Na₂CO₃, KOH, Na₂SO₃ or combination thereof. In another embodiment the base is added at a concentration of between 1-10% w/w to the water to obtain an aqueous base solution.

In another embodiment, the washing step with an aqueous reducing agent is conducted before the washing step with the aqueous base. In another embodiment, the washing step with the aqueous base is conducted before the washing step with the aqueous reducing agent. In another embodiment, the washing step comprises washing with an aqueous reducing agent and a base.

Such a combination of an aqueous reducing agent and a base includes Na₂SO₃ and NaBH₄/NaOH which are basic reducing agents that combine properties of reducing agent and a base.

In another embodiment, the washing steps of this invention are conducted using the organic solvent of the reaction mixture as the organic phase. In another embodiment, the washing step with the aqueous base and the washing step with the aqueous reducing agent are independently performed using a) the organic solvent of the reaction mixture, b) a mixture of organic solvents, or c) a different organic solvent, as the organic phase. Non limiting examples of organic solvents used as an organic phase in the washing step are hydrocarbon solvent, halocarbon solvent, or esters such as cyclohexane, heptane, hexane, pentane, benzene, toluene, chlorobenzene, 1,2-dichloroethane, carbon tetrachloride, 1,3-dichloropropane, 1,2,2-tertrachlorodifluoroethane, 1,2-trichloroethane, trichloroethylene, perchloroethylene, dichloromethane, chloroform, ethyl acetate or butyl acetate.

In one embodiment, following the washing step, the aqueous phase is treated with an acid or an aqueous acid solution to precipitate solid cyanuric acid.

In one embodiment, the organic halide product of the halo-de-carboxylation reaction is soluble in organic phase and not soluble in the aqueous phase. In another embodiment, the crude organic bromide is isolated from reaction mixture by standard organic solvent extractive work-up.

In one embodiment, removing the solvent from the organic phase gives the crude desired halide (**1A**) or (**1B**) (where X is Br or Cl), or a mixture thereof as the residue. In another embodiment, the residue is the pure desired halide (**1A**) or (**1B**). In another embodiment, the residue is the pure desired bromide (**1A**) or (**1B**) (wherein X is Br). In another embodiment, the residue is the pure desired chloride (**1A**) or (**1B**) (wherein X is Cl). In another embodiment, the halide is purified by crystallization, rectification or chromatography of the residue.

In another embodiment the isolation and purification further comprises a drying step. In another embodiment the purification further comprises chromatography.

In one embodiment, the process of this invention provides a process for the preparation of pure organic halide. In another embodiment, the process of this invention provides a process for the preparation of pure organic chloride. In another embodiment, the process of this invention provides a process for the preparation of pure organic bromide. In another embodiment, the process of this invention provides a process for the preparation of mixtures of organic chloride and organic bromide.

In another embodiment, the "pure halide", "pure chloride" or "pure bromide" refers to 92% or more purity. In another embodiment, the "pure halide", "pure chloride" or "pure bromide" refers to about 95% or more purity. In another embodiment, the "pure halide", "pure chloride" or "pure bromide" refers to about 90% or more purity. In another embodiment, the "pure halide", "pure chloride" or "pure bromide" refers to about 85% or more purity. In another embodiment, the "pure halide", "pure chloride" or "pure bromide" refers to about 99% or more purity. In another embodiment, the "pure halide", "pure chloride" or "pure bromide" refers to about 98% or more purity. In another embodiment, the "pure halide", "pure chloride" or "pure bromide" refers to about 97% or more purity

In one embodiment, this application discloses organic halide compounds represented by the formula (**1A**) or (**1B**) having purity of about 99% or more, prepared according to the process of this invention. In another embodiment, this application discloses organic halide compounds represented by the formula (**1A**) or (**1B**) having purity of about 98% or more prepared according to the process of this invention. In another embodiment, this application discloses organic halide compounds represented by the formula (**1A**) or (**1B**) having purity of about 90% or more, prepared according to the process of this invention. In another embodiment, this application discloses organic halide compounds represented by the formula (**1A**) or (**1B**) having purity of about 95% or more, prepared according to the process of this invention. In another embodiment, this application discloses organic halide compounds represented by the formula (**1A**) or (**1B**) having purity of about 85% or more, prepared according to the process of this invention. In another embodiment, this application discloses organic halide compounds represented by the formula (**1A**) or (**1B**) having purity of about 97% or more, prepared according to the process of this invention. In another embodiment, X is Cl. In another embodiment, X is Br.

In one embodiment, the process of this invention, represented by schemes 1 and 2, provides a yield of 60% or more. In another embodiment, the process of this invention provides a yield of 70% or more. In another embodiment, the process of this invention provides a yield of 80% or more. In another embodiment, the process of this invention provides a yield of 85% or more. In another embodiment, the process of this invention provides a yield of 90% or more. In another embodiment, the process of this invention provides a yield of 95% or more.

In one embodiment, this invention is directed to a process and composition comprising carboxylic acid, chloroisocyanurate and brominating agent in a certain molar ratio.

In one embodiment the chloroisocyanurate : (each carboxylic group of said carboxylic acid of formula (2A)) molar ratio is between 0.1 and 2. In another embodiment the chloroisocyanurate : (each carboxylic group of the carboxylic acid of formula **(2A)**) molar ratio is between 1 and 2. In another embodiment the chloroisocyanurate : (each carboxylic group of the carboxylic acid of formula **(2A)**) molar ratio is between 0.1 and 1. In another embodiment the chloroisocyanurate: (each carboxylic group of the carboxylic acid of formula **(2A)**) molar ratio is 1. In another embodiment the chloroisocyanurate: (each carboxylic group of the carboxylic acid of formula **(2A)**) molar ratio is between 1 and 1.5.

In one embodiment, the reaction mixture of the process according to this invention, further comprises a brominating agent. In another embodiment, the brominating agent: (each carboxylic group of the carboxylic acid of formula **(2A)**) molar ration is between 0.1 and 4. In another embodiment, brominating agent: (each carboxylic group of the carboxylic acid of formula **(2A)**) molar ration is between 1 and 4. In another embodiment, the brominating agent: (each carboxylic group of the carboxylic acid of formula **(2A)**) molar ration is between 0.1 and 2. In another embodiment, the brominating agent: (each carboxylic group of the carboxylic acid of formula **(2A)**) molar ration is between 0.1 and 1. In another embodiment the brominating agent: (each carboxylic group of the carboxylic acid of formula **(2A)**) molar ration is between 1 and 2. In another embodiment the brominating agent: ((each carboxylic group of the carboxylic acid of formula **(2A)**) molar ration is between 1 and 3

In one embodiment, this invention is directed to a radiation-sensitive composition comprising a carboxylic acid **(2A)** 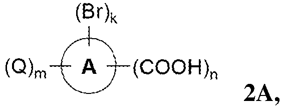 a chloroisocyanurate and a brominating agent which generates organic halide (**1A**) 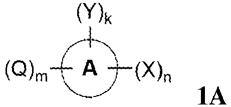 upon electromagnetic irradiation,
wherein
said chloroisocyanurate is trichloroisocyanuric acid, dichloroisocyanuric acid, or any combination thereof;
**A** is arene, branched alkane, cycloalkane or saturated heterocycle;
**n** is an integer greater than or equal to 1;
**X** is Cl or Br; wherein if n > 1, then X may be the same or different;
**k** is an integer greater than or equal to 0;
**Y** is Cl or Br; wherein if k > 1, then Y may be the same or different;
**m** is an integer greater than or equal to 0;
each **Q** is independently F, Cl, Br, R¹, acyl, C(O)R¹, C(O)OR¹, C(O)OMe, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R¹, N(R¹)SO₂R¹, N(acyl)₂, N[C(O)R¹]SO₂R¹, N[C(O)R¹]₂, CF₃; or any two vicinal Q substituents are joined to form a 5- or 6-membered substituted or unsubstituted, saturated or unsaturated carbocyclic or heterocyclic ring;
   wherein each R¹ is independently aryl, alkyl, cycloalkyl or heterocyclyl, wherein R¹ is optionally substituted by one or more substituents of R² ;
   wherein each R² is independently F, Cl, Br, COOH, acyl, aryl, alkyl, cycloalkyl or heterocyclyl;
   wherein if either one of R² in **(2A)** is a carboxylic group COOH, then the respective R² in (**1A**) is Br or Cl;
   wherein the position of said Br and Q in said structure of formula (**1A**) correspond to the same position of said COOH and Q, respectively in said structure of formula **(2A).**

In another embodiment, this invention is directed to a radiation-sensitive composition comprising a carboxylic acid, chloroisocyanurate and a brominating agent; wherein said carboxylic acid is represented by the structure of compound **(2B):** 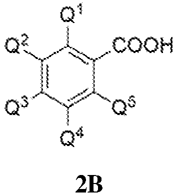 which generates organic halide (**1B**) 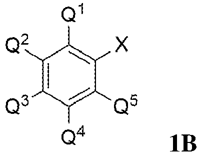 upon electromagnetic irradiation,
wherein
X is Cl or Br;
wherein Q¹, Q², Q³, Q⁴, and Q⁵, are each independently selected from: H, F, Cl, Br, R¹, COOH, acyl, C(O)R¹, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R², N(R¹)SO₂R¹, N(acyl)₂, N[C(O)R¹]SO₂R¹, N[C(O)R¹]₂, CF₃; or any two of Q¹ and Q², Q² and Q³, Q³ and Q⁴ , or Q⁴ and Q⁵, are joined to form a 5- or 6-membered substituted or unsubstituted, saturated or unsaturated carbocyclic or heterocyclic ring;
   wherein each R¹ is independently aryl, alkyl, cycloalkyl or heterocyclyl wherein R¹ is optionally substituted by one or more substituents of R²;
   wherein each R² is independently F, Cl, Br, COOH, acyl, aryl, alkyl, cycloalkyl or heterocyclyl;
   wherein if either one of Q¹, Q², Q³, Q⁴, Q⁵, and/or R² in **(2B)** is carboxylic group COOH, then the respective Q¹, Q², Q³, Q⁴, Q⁵, and/or R² in (**1B**) is Cl or Br;
   wherein if either one of Q¹, Q², Q³, Q⁴, Q⁵, and/or R² in **(2B)** is bromine (Br) then the respective Q¹, Q², Q³, Q⁴, Q⁵, and/or R² in (**1B**) is Cl or Br.

### Mechanism of the halo-de-carboxylation reaction of the invention

Without bounding to any particular mechanism or theory, it is contemplated that the process according to this invention is described as follows:
i. Reaction of the brominating agent with the chloroisocyanurate to yield bromoisocyanurate and chlorinating agent, according to equation (1):

   chloroisocyanurate + brominating agent → bromoisocyanurate + chlorinating agent (1)
ii. Bromination of the carboxylic acid R-CO₂H with the bromoisocyanurate of equation (1) to give the corresponding acyl hypobromite, R-CO₂Br, according to equation (2): 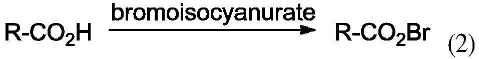
iii. Homolytic degradation of the acyl hypobromite, R-CO₂Br, to give carbon-centered free radical R· according to equation (3):

   R-CO₂Br → R· + CO₂ + Br· (3)
iv. R· pulls out a bromine atom from nearest bromine atom donor to yield bromide R-Br according to equation (4):

   R· + bromine atom donor → R-Br (4)

   wherein the bromine atom donor is selected from: Br· (equation (3)), brominating agents (Br₂, Br₃⁻), or the halocarbon solvent (e.g., BrCCl₃, CF₃CHBrCl).
v. Optionally, chloro-de-bromination of the bromide R-Br with the chlorinating agent (equation 1) takes place to yield the corresponding chloride, R-Cl, and recovery the brominating agent, according to equation (5):

   R-Br + chlorinating agent → R-Cl + brominating agent (5)

Another suggested mechanism for the halo-de-carboxylation of aryl-carboxylic acid according to equation (1a) is represented below: 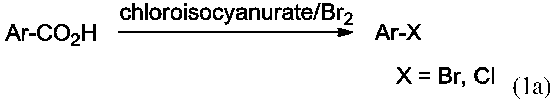
i. Reaction of molecular bromine (brominating agent) with chloroisocyanurate to yield bromine monochloride BrCl (chlorinating agent) and bromoisocyanurate, according to equation (2a):

   chloroisocyanurate + Br₂ → bromoisocyanurate + BrCl (2a)
ii. Bromination of aryl-carboxylic acid Ar-CO₂H with the bromoisocyanurate to give aroyl hypobromite Ar-CO₂Br, according to equation (3a): 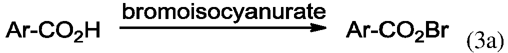
iii. Homolytic degradation of the aroyl hypobromite Ar-CO₂Br to give carbon-centered free radical Ar· according to equation (4a):

   Ar-CO₂Br → Ar· + CO₂ + Br· (4a)
iv. Ar· pulls out a bromine atom from nearest bromine atom donor to yield aryl-bromide Ar-Br according to equation (5a):

   Ar· + bromine atom donor → Ar-Br (5a)

   wherein the bromine atom donor is selected from: Br· (equation (4a)), brominating agents (Br₂ , Br₃⁻), BrCl (equation 2a), or the halocarbon solvent (e.g., BrCCl₃, CF₃CHBrCl);
v. Optionally, radical chloro-de-bromination of the aryl-bromide, Ar-Br, takes place with bromine monochloride (chlorinating agent) to yield aryl-chloride Ar-Cl and recovery of molecular bromine (brominating agent), according to equation (6a):

   Ar-Br + BrCl → Ar-Cl + Br₂ (6a)

A suggested mechanism for the halo-de-carboxylation reaction of alkanoic acid according to equation (1b) is described below: 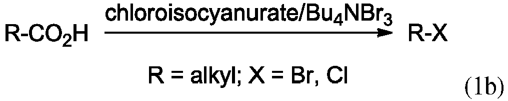
i. Reaction of tetrabutylammonium tribromide (brominating agent) with chloroisocyanurate to yield tetrabutylammonium chloride (chlorinating agent), bromine (brominating agent) and bromoisocyanurate, according to equation (2b)

   chloroisocyanurate + Bu₄NBr₃ → bromoisocyanurate + Bu₄NCl + Br₂ (2b)
ii. Bromination of alkanoic acid R-CO2H with the bromoisocyanurate to give acyl hypobromite R-CO₂Br, according to equation (3b): 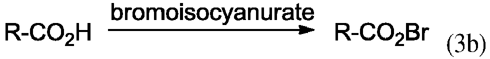
iii. Homolytic degradation of the acyl hypobromite R-CO₂Br to give carbon-centered free radical R· according to equation (4b):

   R-CO₂Br → R· + CO₂ + Br· (4b)
iv. R· pulls out a bromine atom from nearest bromine atom donor to yield alkyl bromide, R-Br, according to equation (5b):

   R· + bromine atom donor → R-Br (5b)
v. Optionally, chloro-de-bromination of the alkyl bromide, R-Br, with tetrabutylammonium chloride (chlorinating agent) takes place to yield alkyl chloride R-Cl, and recovery tetrabutylammonium bromide (brominating agent), according to equation (6b):

   R-Br + Bu₄NCl → R-Cl + Bu₄NBr (6b).

It should be noted that the suggested mechanism presented above, is only a rough scheme of the complex real processes.

One indication for the radical chain mechanism of the halo-de-carboxylation reaction is by using a 2,2,6,6-tetramethyl-1-piperidinynyloxyl (TEMPO) carbon-centered radical scavenger as a mechanistic diagnostic tool. Addition of TEMPO as radical chain inhibitor to the initial reaction mixture of the halo-de-carboxylation reaction, inhibits the reaction. Inhibition of the halo-de-carboxylation reaction by addition of TEMPO indicates that the reaction has a radical chain mechanism.

According to the present invention, the carbon-centered free radicals R· are obtained by applying photochemical and/or thermal energy to a mixture of carboxylic acid R-CO₂H, chlorinating agent (e.g., chloroisocyanurate such as trichloroisocyanuric acid or dichloroisocyanuric acid) and brominating agent. The photochemical energy increases the rate of the reaction.

The term "about" as used herein means within an acceptable error range for the particular value as determined by one of ordinary skill in the art, which will depend in part on how the value is measured or determined, i.e., the limitations of the measurement system. For example, "about" can mean within 1 or more than 1 standard deviations, per the practice in the art. Alternatively, "about" can mean a range of up to about 5%, up to about 10% or up to about 20% of a given value.

The following examples are presented in order to more fully illustrate the preferred embodiments of the invention. They should in no way, however, be construed as limiting the broad scope of the invention.

### EXAMPLES

### Experimental Details:

**Reagents:** All reagents and solvents were purchased from Sigma-Aldrich, Alfa Aesar, Acros Organics, and TCI unless specified otherwise.

**Techniques:** All reactions were performed under nitrogen atmosphere in non-flame dried glassware. Mounted nearby the reaction flask 3 W LED warm-white lamp was used for irradiation of the reaction mixture. Conversions were determined by ¹H NMR, and yields of isolated product refer to products with more than 95% purity by ¹H NMR. Flash column chromatography was performed employing 63-200 µm silica gel 60 according to standard techniques (J. Org. Chem. 1978, v. 43, 2923).

**Analytical methods:** GC analyses were performed on Shimadzu GC-2010 gas chromatograph with flame ionization detector (FID) using a 30 m × 0.25 mm Quadrex capillary column with methyl 5% phenyl silicone stationary phase, 0.25 µm film thickness. For TLC analysis, Merck precoated TLC plates (silica gel 60 F-254 on glass plates, 0.25 mm) were used. NMR spectra were recorded on a Bruker AM-400 (¹H at 400 MHz, ¹³C at 100 MHz) instruments using CDCl₃ (unless otherwise stated) as a solvent. Data are reported as follows: chemical shift in ppm relative to internal TMS, multiplicity, coupling constant in Hz and integration. Compounds described in the literature were characterized by comparing their ¹H and/or ¹³C NMR spectra to the previously reported data. New compounds were further characterized by high-resolution mass spectra.

The following abbreviations are used:
Alk =alkyl
Ar= aryl
CPT = *N*-chlorophthalimide
CTAB = cetyltrimethylammonium bromide
d =doublet
DCCA = dichloroisocyanuric acid
DCE = 1,2-dichloroethane
DCDMH = 1,3-dichloro-5,5-dimethylhydantoin
DCM = dichloromethane
FL = fluorescent room lighting
hv = visible light irradiation
LL = LED lamp irradiation
m = multiplet
*N*-chloroamide = chloroamide, wherein chlorine atom is attached directly to nitrogen atom
NCS = *N*-chlorosuccinimide
NCSac = *N*-chlorosaccharine
NL= dark
rt = room temperature
s = singlet
SDS = sodium dodecyl sulfate
t = triplet
TL = tungsten lamp irradiation
TCCA = trichloroisocyanuric acid
Δ = heating

### EXAMPLE 1

### Chlorodecarboxylation of o-chlorobenzoic acid

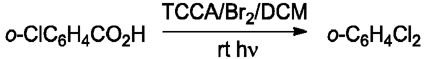

A mixture of o-chlorobenzoic acid (0.16 g, 1 mmol), TCCA (0.24 g, 1 mmol), Br₂ (0.64 g, 4 mmol) and DCM (10 mL) was stirred at rt under fluorescent room light irradiation for 24 h. The reaction mixture was washed with 1 M aq Na₂SO₃, dried over Na₂SO₄, filtered through short neutral alumina pad and concentrated in vacuo. The residue was dissolved in pentane, washed with 1 M aq Na₂SO₃, dried over Na₂SO₄, filtered through short neutral alumina pad and concentrated in vacuo to give o-dichlorobenzene (0.12 g, 80%).

### EXAMPLE 2

### Bromodecarboxylation of 2-nitrobenzoic acid

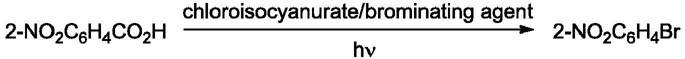

A round bottom flask equipped with Dimroth condenser (chilled to 10 °C) was charged with 2-nitrobenzoic acid (0.6 mmol), chloroisocyanurate, brominating agent and solvent (4 mL). The mixture was magnetically stirred and heated in an oil bath under fluorescent room light irradiation (FL). The cooled reaction mixture was filtered through short silica gel pad, washed with 1 M aq Na₂SO₃, dried over Na₂SO₄, and filtered. The yield of 2-nitrobromobenzene was determined by gas chromatography (GC) using 1,2,4-trichlorobenzene as internal standard. The results of the reactions are presented in Table 1:

**Table 1. Bromodecarboxylation of 2-nitrobenzoic acid ^{a}**

| **entry** | **Reaction conditions** | **yield %^{b}** |
|---|---|---|
| 1 | TCCA 1 mol/Br₂ 2 mol/DCM, 60° FL 18 h | 4 |
| 2 | TCCA 1 mol/Br₂ 2 mol/CHCl₃, 80° FL 18 h | 92 |
| 3 | TCCA 1 mol/Br₂ 1 mol/CCl₄, 100° FL 18 h | 55 |
| 4 | TCCA 1 mol/Br₂ 2 mol/CCl₄, 100° FL 18 h | 96 |
| 5^{c} | TCCA 1 mol/Br₂ 2 mol/CCl₄, 100° FL 18 h | 20 |
| 6 | TCCA 1 mol/Br₂ 2 mol/CBrCl₃, 120° FL 18 h | 97 |
| 7^{c} | TCCA 1 mol/Br₂ 2 mol/CBrCl₃, 120° FL 18 h | 80 |

| | | |
|---|---|---|
| ^{a} All quantities in mole/mole of 2-nitrobenzoic acid. Oil bath temperatures in degrees Celsius. ^{b} Yield is based on 2-nitrobromobenzene analyzed by GC. ^{c} The reaction was provided in the absence of light. | | |

### EXAMPLE 3

### Bromodecarboxylation of 2-nitrobenzoic acid, 57 mmol scale-up.

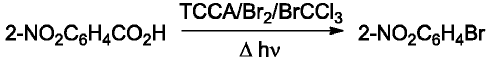

A 250 mL round bottom flask equipped with Dimroth condenser (chilled to 10 °C) was charged with 2-nitrobenzoic acid (57.1 mmol), TCCA (57.1 mmol), Br₂ (114 mmol) and BrCCl₃ (95 mL). The mixture was stirred and heated in an oil bath at 120 °C under fluorescent room light irradiation for 18 h. The cooled reaction mixture was filtered through short silica gel pad, washed with 1 M aq Na₂SO₃, dried over Na₂SO₄, filtered and concentrated *in vacuo* to obtain 10.6 g (92%) of 2-nitrobromobenzene.

### EXAMPLE 4

### Bromodecarboxylation of nitroarenecarboxylic acids

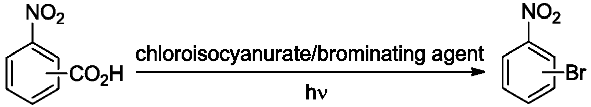

A 25 mL round bottom flask equipped with Dimroth condenser (chilled to 10 °C) was charged with nitroarenecarboxylic acid ArCO₂H (1.8 mmol), chloroisocyanurate, brominating agent and solvent (8 mL). The mixture was stirred and heated in an oil bath under fluorescent room light irradiation (FL). The cooled reaction mixture was filtered through short silica gel pad, washed with 1 M aq Na₂SO₃, dried over Na₂SO₄, filtered and concentrated *in vacuo* to obtain bromonitroarene ArBr. The obtained product contained between 1-5% of the corresponding chloronitroarene ArCl as a by-product. The results are presented in Table 2.

**Table 2. Bromodecarboxylation of nitroarenecarboxylic acids ArCO₂H ^{a}**

| **entry** | **ArCO₂H** | **Reaction conditions** | **yield % ^{b}** |
|---|---|---|---|
| 1 | 2-NO₂C₆H₄CO₂H | TCCA 1 mol/Br₂ 2 mol/ CCl₄, 100° FL 18 h | 92 |
| 2 | 3-Cl-2-NO₂C₆H₃CO₂H | TCCA 1 mol/Br₂ 2 mol/ CBrCl₃, 120° FL 18 h | 67^{c} |
| 3 | 4-Cl-2-NO₂C₆H₃CO₂H | TCCA 1 mol/Br₂ 2 mol/ CCl₄, 100° FL 18 h | 96 |
| 4 | 5-Cl-2-NO₂C₆H₃CO₂H | TCCA 1 mol/Br₂ 2 mol/ CCl₄, 100° FL 18 h | 95 |
| 5 | 4-Br-2-NO₂C₆H₃CO₂H | TCCA 1 mol/Br₂ 2 mol/ CCl₄, 100° FL 18 h | 97 |
| 6 | 2,4-(NO₂)₂C₆H₃CO₂H | TCCA 1 mol/Br₂ 2 mol/ CBrCl₃, 120° FL 18 h | 91 |
| 7 | 3-NO₂C₆H₄CO₂H | TCCA 1 mol/Br₂ 2 mol/ CCl₄, 100° FL 18 h | 93 |
| 8 | 4-F-3-NO₂C₆H₃CO₂H | TCCA 1 mol/Br₂ 2 mol/ CBrCl₃, 120° FL 18 h | 73^{c} |
| 9 | 2-Cl-3-NO₂C₆H₃CO₂H | TCCA 1 mol/Br₂ 2 mol/ CBrCl₃, 120° FL 18 h | 85^{c} |
| 10 | 4-Cl-3-NO₂C₆H₃CO₂H | TCCA 1 mol/Br₂ 2 mol/ CBrCl₃, 120° FL 18 h | 84^{c} |
| 11 | 2,5-Cl₂-3-NO₂C₆H₂CO₂H | TCCA 1 mol/Br₂ 2 mol/ CBrCl₃, 120° FL 18 h | 92 |
| 12 | 4-NO₂C₆H₄CO₂H | TCCA 1 mol/Br₂ 2 mol/ CBrCl₃, 120° FL 18 h | 84^{c} |
| 13 | 3-CF₃-4-NO₂C₆H₃CO₂H | TCCA 1 mol/Br₂ 2 mol/ CCl₄/CHCl₃^{d}, 100° FL 18 h | 79 |
| 14 | 2-F-4-NO₂C₆H₃CO₂H | TCCA 1 mol/Br₂ 2 mol/ CCl₄, 100° FL 18 h | 84^{c} |
| 15 | 2-Cl-4-NO₂C₆H₃CO₂H | TCCA 1 mol/Br₂ 2 mol/ CC14/CHC1₃^{d}, 100° FL 18 h | 94 |

| | | | |
|---|---|---|---|
| ^{a} All quantities in mole/mole of nitroarenecarboxylic acid. Oil bath temperatures in degrees Celsius. ^{b} Isolated yield of bromonitroarenes ArBr. ^{c} The yield was determined after purification of the product by chromatography on silica gel. ^{d} Mixture of CCl₄ and CHCl₃ 4:1 v/v was used as solvent. | | | |

**Entry 1: 1-bromo-2-nitrobenzene** ¹H NMR: δ 7.85-7.8 (m, 1H), 7.75-7.7 (m, 1H), 7.5-7.4 (m, 2H) ppm; ¹³C NMR: δ 149.8, 135.1, 133.3, 128.4, 125.6, 114.4 ppm.
**Entry 2: 1-bromo-2-chloro-3-nitrobenzene** ¹H NMR: δ 7.84 (dd, *J* = 8, 1 Hz, 1H), 7.72 (dd, *J* = 8, 1 Hz, 1H), 7.30 (t, *J* = 8 Hz, 1H) ppm; ¹³C NMR δ 149.7, 137.0, 128.2, 127.2, 125.5, 123.9 ppm.
**Entry 3: 1-bromo-4-chloro-2-nitrobenzene** ¹H NMR δ 7.81 (d, *J* = 2 Hz, 1H), 7.67 (d, *J* = 9 Hz, 1H), 7.41 (dd, *J* = 9, 2 Hz, 1H)ppm; ¹³C NMR: δ 150, 136, 134.3, 133.4, 125.7, 112.5 ppm.
**Entry 4: 2-bromo-4-chloro-1-nitrobenzene** ¹H NMR δ 7.83 (d, *J* = 9 Hz, 1H), 7.71 (d, *J* = 2 Hz, 1H), 7.44 (dd, *J* = 9, 2 Hz, 1H) ppm; ¹³C NMR: δ 148, 139.2, 134.7, 128.5, 126.7, 115.5 ppm.
**Entry 5: 1,4-dibromo-2-nitrobenzene** ¹H NMR δ 7.94 (d, *J* = 2 Hz, 1H), 7.59 (d, *J* = 9 Hz, 1H), 7.54 (dd, *J* = 9, 2 Hz, 1H) ppm; ¹³C NMR: δ 150, 136.3, 136.2, 128.5, 121.5, 113.2 ppm.
**Entry 6: 1-bromo-2,4-dinitrobenzene** ¹H NMR: δ 8.64 (d, *J* = 3 Hz, 1H), 8.29 (dd, *J* = 9, 3 Hz, 1H), 8.02 (d, 1H, *J* = 9 Hz) ppm; ¹³C NMR δ 149.7, 147.0, 136.6, 127.3, 121.9 ppm.
**Entry 7: 1-bromo-3-nitrobenzene** ¹H NMR: δ 8.29 (t, *J* = 2 Hz, 1H), 8.13 (ddd, *J* = 8, 2, 1 Hz, 1H), 7.81 (ddd, *J* = 8, 2, 1 Hz, 1H), 7.44 (t, *J* = 8 Hz, 1H) ppm; ¹³C NMR: δ 148.6, 137.5, 130.6, 126.5, 122.7, 122.1 ppm.
**Entry 8: 4-bromo-1-fluoro-2-nitrobenzene** ¹H NMR: δ 8.16 (dd, *J* = 7, 2 Hz, 1H), 7.76 (ddd, *J* = 9, 4, 3 Hz, 1H), 7.23 (dd, *J* = 10, 9 Hz, 1H) ppm; ¹³C NMR: δ 154.6 (d, *J_{CF}* = 265.9), 138.6 (d, *J_{CF}* = 8 Hz), 137.8, 128.8 (d, *J_{CF}* = 3 Hz), 120.2 (d, *J_{CF}* = 22 Hz), 116.7 (d, *J_{CF}* = 4 Hz) ppm. ¹⁹F NMR: δ -122.2 ppm.
**Entry 9: 1-bromo-3-chloro-2-nitrobenzene** ¹H NMR: δ 7.58 (dd, *J* = 8, 1 Hz, 1H), 7.47 (dd, *J* = 8, 1 Hz, 1H), 7.32 (t, *J* = 8 Hz, 1H) ppm; ¹³C NMR: δ 150.0, 132.15, 131.6, 129.7, 126.3, 113.9 ppm.
**Entry 10: 4-bromo-1-chloro-2-nitrobenzene** ¹H NMR δ 8.00 (d, *J* = 2 Hz, 1H), 7.64 (dd, *J* = 9, 2 Hz, 1H), 7.43 (d, *J* = 9 Hz, 1H) ppm; ¹³C NMR δ 148.3, 136.2, 133.1, 128.5, 126.2, 120.7 ppm.
**Entry 11: 1-bromo-2,5-dichloro-3-nitrobenzene** ¹H NMR: δ 7.83 (d, *J* = 2 Hz, 1H), 7.72 (d, *J* = 2 Hz, 1H) ppm; ¹³C NMR δ 149.4, 136.5, 133.7, 126.07, 126.06, 124.2 ppm.
**Entry 12: 1-bromo-4-nitrobenzene** ¹H NMR: δ 8.08 (d, *J* = 9 Hz, 1H), 7.67 (d, *J* = 9 Hz, 1H) ppm; ¹³C NMR: δ 147.1, 132.7, 130.1, 125.1 ppm.
**Entry 13: 4-bromo-1-nitro-2-(trifluoromethyl)benzene** ¹H NMR: δ 8.5 (d, *J* = 2 Hz, 1H), 8.26 (dd, *J* = 9, 2 Hz, 1H), 7.96 (d, *J* = 9 Hz, 1H) ppm; ¹³C NMR δ 146.9, 136.5, 131.8 (q, *J_{CF}* = 33 Hz), 127.7 (d, *J_{CF}* = 1 Hz), 127.5, 123.2 (q, *J_{CF}* = 6 Hz), 122 (q, *J_{CF}* = 274 Hz) ppm; ¹⁹F NMR: δ -66.4 ppm.
**Entry 14: 1-bromo-2-fluoro-4-nitrobenzene** ¹H NMR δ 7.99-7.91 (m, 2H), 7.78 (dd, *J* = 9, 7 Hz, 1H) ppm; ¹³C NMR: δ 158 (d, *J_{CF}* = 252 Hz), 147.9 (d, *J_{CF}* = 7 Hz), 134.3, 120.2 (d, *J_{CF}* = 4 Hz), 117.3 (d, *J_{CF}* = 21 Hz), 112.1 (d, *J_{CF}* = 27 Hz) ppm; ¹⁹F NMR δ -105.0 ppm.
**Entry 15: 1-bromo-2-chloro-4-nitrobenzene** ¹H NMR: δ 8.26 (d, *J* = 2 Hz, 1H), 7.97 (dd, *J* = 9, 2 Hz, 1H), 7.80 (d, *J* = 9 Hz, 1H) ppm; ¹³C NMR: δ 147.3, 135.8, 134.4, 130.3, 125.1, 122.5 ppm.

### EXAMPLE 5

### Halo-de-carboxylation of 4-methoxy-2-nitrobenzoic acid

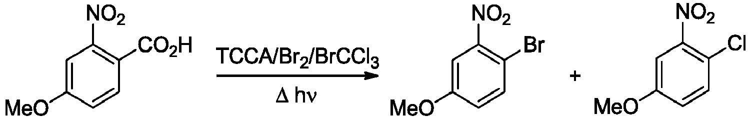

A 25 mL round bottom flask equipped with Dimroth condenser (chilled to 10 °C) was charged with 4-methoxy-2-nitrobenzoic acid (304 mg, 1.54 mmol), TCCA (357 mg, 1.54 mmol), Br₂ (492 mg, 3.08 mmol) and BrCCl₃ (8 mL). The mixture was stirred and heated in an oil bath at 120 °C under fluorescent room light irradiation for 18 h. The cooled reaction mixture was filtered through short silica gel pad, washed with 1 M aq Na₂SO₃, dried over Na₂SO₄, filtered and concentrated *in vacuo.* The residue was purified by chromatography on silica gel (eluent hexane/DCM) to give the mixture of 4-bromo- and 4-chloro-3-nitroanisoles (250 mg, 82:18 by GC).

### EXAMPLE 6

### Bromodecarboxylation of nitroarenedicarboxylic acids

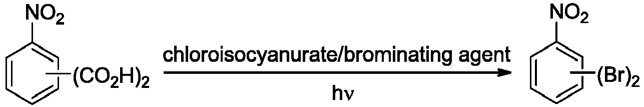

Round bottom flask equipped with Dimroth condenser (chilled to 10 °C) was charged with nitroarenedicarboxylic acid NO₂C₆H₃(CO₂H)₂ (0.95 mmol), chloroisocyanurate, brominating agent and solvent (8 mL). The mixture was stirred and heated in an oil bath at 120 °C under fluorescent room light irradiation. The cooled reaction mixture was filtered through short silica gel pad, washed with 1 M aq Na₂SO₃, dried over Na₂SO₄, filtered and concentrated *in vacuo.* The obtained dibromonitroarene NO₂C₆H₃Br₂ is 95-99% pure (by GC) and may contain 1-5% of corresponding monochlorinated product NO₂C₆H₃BrCl. The results are presented in Table 3.

**Table 3. Bromodecarboxylation of nitroarenedicarboxylic acids NO₂C₆H₃(CO₂H)₂ ^{a}**

| **entry** | **NO₂C₆H₃(CO₂H)₂** | **Reaction conditions** | **yield % ^{b}** |
|---|---|---|---|
| 1 | 3-NO₂-1,2-C₆H₃(CO₂H)₂ | TCCA 2 mol/Br₂ 4 mol/ CBrCl₃, 120° FL 60 h | 85 |
| 2 | 4-NO₂-1,2-C₆H₃(CO₂H)₂ | TCCA 2 mol/Br₂ 4 mol/ CBrCl₃, 120° FL 60 h | 66 |
| 3 | 5-NO₂-1,3-C₆H₃(CO₂H)₂ | TCCA 2 mol/Br₂ 4 mol/ CBrCl₃, 120° FL 60 h | 82 |

| | | | |
|---|---|---|---|
| ^{a} All quantities in mole/mole of nitroarenedicarboxylic acid. Oil bath temperatures in degrees Celsius. ^{b} Isolated yield of dibromonitroarenes NO₂C₆H₃Br₂ | | | |

**Entry 1: 1,2-dibromo-3-nitrobenzene** ¹H NMR: δ 7.84 (d, *J* = 8 Hz, 1H), 7.63 (d, *J* = 8 Hz, 1H), 7.33 (t, *J* = 8 Hz, 1H) ppm; ¹³C NMR: δ 152.1, 136.6, 129.0, 128.0, 123.5, 117.3 ppm.
**Entry 2: 1,2-dibromo-4-nitrobenzene** ¹H NMR: δ 8.42 (d, *J* = 2 Hz, 1H), 8.00 (dd, *J* = 9, 2 Hz, 1H), 7.80 (d, *J* = 9 Hz, 1H) ppm; ¹³C NMR: 147.2, 134.3, 132.8, 128.5, 125.8, 123.1 ppm.
**Entry 3: 1,3-dibromo-5-nitrobenzene** ¹H NMR: δ 8.30 (s, 2H), 8.00 (s, 1H) ppm; ¹³C NMR: δ149.0, 140.0, 125.6, 123.5 ppm.

### EXAMPLE 7

### Disruption of the halo-de-carboxylation in the bromination stage

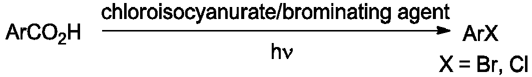

Round bottom flask equipped with Dimroth condenser (chilled to 10 °C) was charged with arenecarboxylic acid ArCO₂H (0.95 mmol), chloroisocyanurate, brominating agent and solvent (8 mL). The mixture was stirred and heated in an oil bath under fluorescent room light irradiation (FL). The cooled reaction mixture was filtered through short silica gel pad, washed with 1 M aq Na₂SO₃, dried over Na₂SO₄, filtered and concentrated *in vacuo.* The residue was characterized by ¹H and ¹³C NMR. The results are presented in Table 4.

**Table 4. Interruption of the halo-de-carboxylation on stage of bromination ^{a}**

| **entry** | **Reaction conditions** | **yields, % ArBr/ArCl** |
|---|---|---|
| 1 | 3-NCC₆H₄CO₂H/TCCA 1 mol/Br₂ 2 mol/ BrCCl₃, FL 120° 0.5 h | 84 : 10 |
| 2 | 4-NCC₆H₄CO₂H/TCCA 1 mol/Br₂ 2 mol/ BrCCl₃, FL 120° 0.5 h | 71 : 6 |
| 3 | 2-ClC₆H₄CO₂H/TCCA 1 mol/Br₂ 2 mol/ BrCCl₃, FL 120° 0.5 h | 69 : 28 |
| 4 | 2,4-Cl₂C₆H₃CO₂H/TCCA 1 mol/Br₂ 2 mol/ BrCCl₃, FL 120° 0.5 h | 72 : 24 |
| 5 | 2,4,6-Cl₃C₆H₂CO₂H/TCCA 1 mol/Br₂ 2 mol/ BrCCl₃, FL 120° 0.5 h | 75 : 22 |

| | | |
|---|---|---|
| ^{a} All quantities in mole/mole of arenecarboxylic acid. Oil bath temperatures in degrees Celsius. | | |

### EXAMPLE 8

### Radical chlorodecarboxylation of benzoic acid induced by N-chloroamide and brominating agent

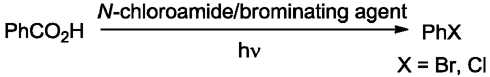

Glass pressure tube filled with benzoic acid (0.8 mmol), *N*-chloroamide, brominating agent and solvent (4 mL) was magnetically stirred and heated in an oil bath under fluorescent room light irradiation (FL). The yield of chlorobenzene was determined by gas chromatography (GC) using 1-chloro-2-fluorobenzene as internal standard. The results are presented in Table 5.

**Table 5. N-Chloroamides as reagents for chlorodecarboxylation of benzoic acid ^{a}**

| **entry** | **Reaction conditions** | **yield %** ^{b} |
|---|---|---|
| 1 | TCCA 1 mol/Br₂ 1 mol/CCl₄, FL 100° 6 h | 92 |
| 2 | DCCA 1 mol/Br₂ 1 mol/CCl₄, FL 100° 6 h | 89 |
| 3 | NCS 1 mol/Br₂ 1 mol/CCl₄, FL 100° 6 h | 0 |
| 4 | DCDMH 1 mol/Br₂ 1 mol/CCl₄, FL 100° 6 h | 7 |
| 5 | CPT 1 mol/Br₂ 1 mol/CCl₄, FL 100° 6 h | 1 |
| 6 | NCSac 1 mol/Br₂ 1 mol/CCl₄, FL 100° 6 h | 0 |

| | | |
|---|---|---|
| ^{a} All quantities in mole/mole of benzoic acid. Oil bath temperatures in degrees Celsius. DCDMH is 1,3-dichloro-5,5-dimethylhydantoin. CPT is *N*-chlorophthalimide. NCSac is *N*-chlorosaccharin. ^{b} Chlorobenzene analyzed by GC. | | |

### EXAMPLE 9

### Chlorodecarboxylation of benzoic acid. Optimization of the reaction conditions.

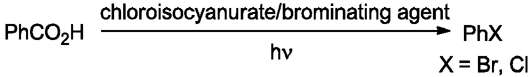

Round bottom flask equipped with Dimroth condenser (chilled to -10 °C) was charged with benzoic acid (0.8 mmol), chloroisocyanurate, brominating agent and solvent (4 mL). The mixture was magnetically stirred and heated in an oil bath under fluorescent room light irradiation (FL). The yields of chloro- and bromobenzenes were determined by gas chromatography (GC) using 1-chloro-2-fluorobenzene as internal standard. The results are presented in Table 6.

**Table 6. Halo-de-carboxylation of benzoic acid ^{a}**

| **Entry** | **Reaction conditions** | **GC yield, % PhCl/PhBr** |
|---|---|---|
| 1^{b} | TCCA 1 mol/Br₂ 3 mol/DCM, FL 60° 3 h | 67 : 0 |
| 2^{b} | TCCA 1 mol/Br₂ 3 mol/CHCl₃, FL 80° 3 h | 82 : 0 |
| 3^{b} | TCCA 1 mol/Br₂ 3 mol/CCl₄, FL 100° 4 h | 87 : 7 |
| 4^{b} | TCCA 1 mol/Br₂ 3 mol/CCl₄, FL 100° 5 h | 91 : 3 |
| 5^{b} | TCCA 1 mol/Br₂ 3 mol/CCl₄, FL 100° 6 h | 92 : 0 |
| 6^{b} | TCCA 1 mol/Br₂ 1 mol/CCl₄, FL 100° 6 h | 93 : 0 |
| 7^{b} | TCCA 1 mol/Br₂ 0.5 mol/CCl₄, FL 100° 6 h | 74 : 1 |
| 8 | TCCA 1 mol/Br₂ 1 mol/CCl₄, FL 100° 6 h | 75 : 13 |
| 9 | TCCA 1 mol/Br₂ 1 mol/CCl₄, FL 100° 6 h | 85 : 6 |
| 10 | TCCA 1 mol/Br₂ 1 mol/CCl₄, FL 100° 21 h | 92 : 0 |
| 11 | TCCA 1 mol/Br₂ 0.75 mol/CCl₄, FL 100° 21 h | 92 : 0 |
| 12 | TCCA 1 mol/Br₂ 0.6 mol/CCl₄, FL 100° 21 h | 90 : 0 |
| 13 | TCCA 1 mol/Br₂ 0.5 mol/CCl₄, FL 100° 21 h | 87 : 0 |
| 14 | TCCA 1 mol/Br₂ 0.3 mol/CCl₄, FL 100° 21 h | 30 : 0 |
| 15 | TCCA 1 mol/CCl₄, FL 100° 21 h | 0 : 0 |

| | | |
|---|---|---|
| ^{a} All quantities in mole/mole of benzoic acid. Oil bath temperatures in degrees Celsius. ^{b} Reaction carried out in glass pressure tube. | | |

### EXAMPLE 10

### Exploring the scope of chlorodecarboxylation reaction of arenecarboxylic acids

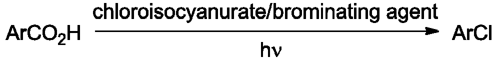

Round bottom flask equipped with Dimroth condenser (chilled to -10 °C) was charged with arenecarboxylic acid (1.8 mmol), chloroisocyanurate, brominating agent and solvent (8 mL). The mixture was magnetically stirred and heated in an oil bath under 3 W LED warm-white lamp irradiation (LL) or under fluorescent room light irradiation (FL). The cooled reaction mixture was filtered through a short silica gel pad, washed with 1 M aq Na₂SO₃, dried over Na₂SO₄, filtered and the solvent was removed by distillation. In case of volatile product the yield was determined by gas chromatography (GC) with internal standard. The results are presented in Table 7.

**Table 7. Chlorodecarboxylation of arenecarboxylic acids ArCO₂H ^{a}**

| **Entry** | **ArCO₂H** | **Reaction conditions** | **yield, % ArCl** |
|---|---|---|---|
| 1 | PhCO₂H | TCCA 1 mol/Br₂ 1 mol/CCl₄, 100° FL 18 h | 92^{c} |
| 2^{b} | 2-FC₆H₄CO₂H | TCCA 1 mol/Br₂ 1 mol/CCl₄, 100° FL 18 h | 82^{c} |
| 3 | C₆F₅CO₂H | TCCA 1 mol/Br₂ 1 mol/CCl₄, 100° LL 18 h | 75^{c} |
| 4 | 4-Cl-2-FC₆H₃CO₂H | TCCA 1 mol/Br₂ 1 mol/CCl₄, 100° LL 18 h | 92 |
| 5 | 3-CF₃-2-FC₆H₃CO₂H | TCCA 1 mol/Br₂ 1 mol/CCl₄, 100° LL 18 h | 74 |
| 6 | 2-CF₃-5-FC₆H₃CO₂H | TCCA 1 mol/Br₂ 1 mol/CCl₄, 100° LL 18 h | 61 |
| 7^{b} | 3-FC₆H₄CO₂H | TCCA 1 mol/Br₂ 1 mol/CCl₄, 100° FL 18 h | 89^{c} |
| 8 | 5-CF₃-3-FC₆H₃CO₂H | TCCA 1 mol/Br₂ 1 mol/CCl₄, 100° LL 18 h | 59 |
| 9^{b} | 4-FC₆H₄CO₂H | TCCA 1 mol/Br₂ 1 mol/CCl₄, 100° FL 18 h | 92^{c} |
| 10^{b} | 2-CF₃C₆H₄CO₂H | TCCA 1 mol/Br₂ 1 mol/CCl₄, 100° LL 18 h | 87^{c} |
| 11^{b} | 2,6-(CF₃)₂C₆H₃CO₂H | TCCA 1 mol/Br₂ 1 mol/CCl₄, 100° LL 18 h | 75 |
| 12^{b} | 3-CF₃C₆H₄CO₂H | TCCA 1 mol/Br₂ 1 mol/CCl₄, 100° FL 18 h | 100^{c} |
| 13 | 3,5-(CF₃)₂C₆H₃CO₂H | TCCA 1 mol/Br₂ 1 mol/CCl₄, 100° LL 18 h | 92 |
| 14^{b} | 4-CF₃C₆H₄CO₂H | TCCA 1 mol/Br₂ 1 mol/CCl₄, 100° LL 18 h | 100^{c} |
| 15^{b} | 2-ClC₆H₄CO₂H | TCCA 1 mol/Br₂ 1 mol/CCl₄, 100° LL 18 h | 87 |
| 16^{b} | 2,4-Cl₂C₆H₃CO₂H | TCCA 1 mol/Br₂ 1 mol/CCl₄, 100° FL 18 h | 97 |
| 17 | 2,4,6-Cl₃C₆H₂CO₂H | TCCA 1 mol/Br₂ 1 mol/CCl₄, 100° LL 18 h | 97 |
| 18^{b} | 3-ClC₆H₄CO₂H | TCCA 1 mol/Br₂ 1 mol/CCl₄, 100° LL 18 h | 78 |
| 19 | 3,4-Cl₂C₆H₃CO₂H | TCCA 1 mol/Br₂ 1 mol/CCl₄, 100° LL 18 h | 93 |
| 20 | 4-ClC₆H₄CO₂H | TCCA 1 mol/Br₂ 1 mol/CCl₄, 100° LL 18 h | 97 |
| 21 | 3-NCC₆H₄CO₂H | TCCA 1 mol/Br₂ 1 mol/CCl₄, 100° LL 18 h | 93 |
| 22 | 4-NCC₆H₄CO₂H | TCCA 1 mol/Br₂ 1 mol/CCl₄, 100° LL 18 h | 94 |
| 23 | trimellitic anhydride | TCCA 1 mol/Br₂ 1 mol/CCl₄, 100° LL 18 h | 86 |

| | | | |
|---|---|---|---|
| ^{a} All quantities in mole/mole of benzoic acid. Oil bath temperatures in degrees Celsius. ^{b} Reaction carried out in glass pressure tube. ^{c} Products analyzed by GC. | | | |

**Entry 4: 1,4-dichloro-2-fluorobenzene** ¹H NMR: δ 7.30 (m, 1H), 7.15 (dd, 1H, *J* = 9, 2 Hz), 7.43 (d, 1H, *J* = 2 Hz), 7.10 (m, 1H) ppm; ¹³C NMR: δ 158.0 (d, *J_{CF}* = 252 Hz), 133.3 (d, *J_{CF}* = 9 Hz), 131.2, 125.2 (d, *J_{CF}* = 4 Hz), 119.8 (d, *J_{CF}* = 18 Hz), 117.4 (d, *J_{CF}* = 24 Hz) ppm; ¹⁹F NMR: δ -115.3 ppm.
**Entry 5: 1-chloro-2-fluoro-3-(trifluoromethyl)benzene** ¹H NMR: δ 761 (m, 1H), 7.52 (m, 1H), 7.20 (m, 1H) ppm; ¹³C NMR δ 155.7 (dq, *J_{CF}* = 259, 2 Hz), 134.6, 125.6 (m), 124.7 (d, *J_{CF}* = 5 Hz), 123.0 (d, *J_{CF}* = 17 Hz), 122.3 (qd, *J_{CF}* = 273, 1.5 Hz), 120.2 (qd, *J_{CF}* = 33, 12 Hz) ppm; ¹⁹F NMR: δ -64.7 (d, *J_{FF}* = 13 Hz), -119.0 (q, *J_{FF} =* 13 Hz) ppm.
**Entry 6: 2-chloro-4-fluoro-1-(trifluoromethyl)benzene** ¹H NMR: δ 7.69 (dd, 1H, *J* = 9, 6 Hz), 7.25 (dd, 1H, *J* = 8, 2 Hz), 7.07 (m, 1H) ppm; ¹³C NMR: δ 164.4 (d, *J_{CF}* = 256 Hz), 134.3 (m), 129.5 (dq, *J_{CF}* = 11, 5 Hz), 124.8 (qd, *J_{CF}* = 32, 4 Hz), 122.8 (q, *J_{CF}* =273 Hz), 119.3 (d, *J_{CF}* = 25 Hz), 114.1 (d, *J_{CF}* = 22 Hz) ppm; ¹⁹F NMR δ -65.2, -108.8 ppm.
**Entry 8: 1-chloro-3-fluoro-5-(trifluoromethyl)benzene** ¹H NMR: δ 7.43 (s, 1H), 7.29 (m, 1H), 7.25 (m, 1H) ppm; ¹³C NMR: δ 162.7 (d, *J_{CF}* = 253 Hz), 136.4 (d, *J_{CF}* = 10 Hz), 133.8 (qd, *J_{CF}* = 34, 9 Hz), 122.8 (qd, *J_{CF}* = 273, 4 Hz), 121.9 (dq, *J_{CF}* = 4, 4 Hz), 119.9 (d, *J_{CF}* = 24 Hz), 111.7 (dq, *J_{CF}* = 25, 4 Hz) ppm; ¹⁹F NMR: δ -66.2, -111.3 ppm.
**Entry 11: 2-chloro-1,3-bis(trifluoromethyl)benzene** ¹H NMR: δ 7.90 (d, 2H, *J* = 8 Hz), 7.51 (t, 1H, *J* = 8 Hz) ppm; ¹³C NMR: δ 131.7 (m), 131.1 (q, *J_{CF}* = 32 Hz), 131.0 (q, *J_{CF}* = 5 Hz), 127.0, 122.5 (q, *J_{CF}* = 274 Hz) ppm; ¹⁹F NMR: δ -66.5 ppm.
**Entry 13: 1-chloro-3,5-bis(trifluoromethyl)benzene** ¹H NMR: δ 781 (s, 2H), 7.78 (s, 1H) ppm; ¹³C NMR: δ 136.2, 133.4 (q, *J_{CF}* = 34 Hz), 129.3 (d, *J_{CF}* = 3 Hz), 122.7 (q, *J_{CF}* = 273 Hz), 120.9 (sept, *J_{CF}* = 4 Hz) ppm; ¹⁹F NMR: δ -66.2 ppm.
**Entry 15: 1,2-dichlorobenzene** ¹H NMR: δ 7.40 (dd, 2H, *J* = 6, 4 Hz), 7.19 (dd, 2H, J = 6, 4 Hz) ppm; ¹³C NMR: δ 132.6, 130.6, 127.8 ppm.
**Entry 16: 1,2,4-trichlorobenzene** ¹H NMR: δ 7.43 (d, 1H, *J* = 2 Hz), 7.34 (d, 1H, *J* = 9 Hz), 7.16 (dd, 1H, *J* = 9, 2 Hz) ppm; ¹³C NMR: δ 133.5, 133.1, 131.2, 131.1, 130.3, 128.0 ppm.
**Entry 17: 1,2,3,5-tetrachlorobenzene** ¹H NMR: δ 7.35 (s, 2H) ppm; ¹³C NMR: δ 134.8, 132.8, 130.4, 128.7 ppm.
**Entry 18: 1,3-dichlorobenzene** ¹H NMR: δ 7.26-7.12 (m, 1H), 7.13-7.09 (m, 3H) ppm; ¹³C NMR: δ 135.1, 130.6, 128.8, 127.0 ppm.
**Entry 20: 1,4-dichlorobenzene** ¹H NMR: δ 7.27 (s, 1H); ¹³C NMR δ 132.6, 129.9 ppm.
**Entry 21: 3-chlorobenzonitrile** ¹H NMR: δ 7.64-7.61 (m, 1H), 7.60-7.56 (m, 1H), 7.56-7.53 (m, 1H), 7.42 (t, 1H, *J* = 8 Hz) ppm; ¹³C NMR: δ 134.9, 133.1, 131.7, 130.4, 130.2, 117.3, 113.8 ppm.
**Entry 22: 4-chlorobenzonitrile** ¹H NMR: δ 7.59 (d, 1H, *J* = 9 Hz), 7.43 (d, 1H, *J* = 9 Hz) ppm; ¹³C NMR: δ 139.2, 133.2, 129.5, 117.8, 110.6 ppm.
**Entry 23: 4-chlorophthalic anhydride** ¹H NMR: δ 7.99 (d, 1H, *J* = 8 Hz), 7.96 (s, 1H), 7.90 (d, 1H, *J* = 8 Hz) ppm; ¹³C NMR: δ 161.7, 161.5, 143.0, 136.5, 132.8, 129.3, 126.9, 125.8 ppm.

### EXAMPLE 11

### Chlorodecarboxylation of o-bromobenzoic acid

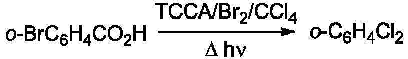

Glass pressure tube filled with *o*-bromobenzoic acid (1.8 mmol), TCCA (3.6 mmol), Br₂ (3.6 mmol) and CCl₄ (8 mL) was magnetically stirred and heated in an oil bath at 100 °C under LED warm-white lamp irradiation for 18 h. The cooled reaction mixture was filtered through a short silica gel pad, washed with 1 M aq Na₂SO₃, dried over Na₂SO₄, filtered and the solvent was removed by distillation to give *o*-dichlorobenzene with 95% yield and 95% purity by GC.

### EXAMPLE 12

### Halo-de-carboxylation of lauric acid

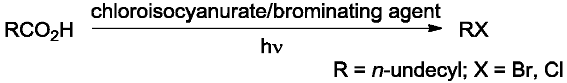

A mixture of lauric acid (0.5 mmol), chloroisocyanurate, brominating agent and solvent (4 mL) was stirred under fluorescent room light irradiation (FL). An aliquot of the reaction mixture washed with 1 M aq Na₂SO₃, dried over Na₂SO₄, and filtered through short neutral silica gel pad. The yields of 1-bromo- and 1-chloroundecanes were determined by gas chromatography (GC) using 1,2,4,5-tetrachlorobenzene as internal standard. The results are presented in Table 8.

**Table 8. Halo-de-carboxylation of lauric acid RCO₂H (R is n-undecyl)^{a}**

| **entry** | **Reaction conditions** | **GC yields, % RBr/RCl** |
|---|---|---|
| 1 | DCCA 1 mol/[NBu₄]Br₃ 2 mol/DCM, FL rt 1 h | 29 : 3 |
| 2 | DCCA 1 mol/[NBu₄]Br₃ 2 mol/DCM, FL rt 2 h | 54 : 5 |
| 3 | DCCA 1 mol/[NBu₄]Br₃ 2 mol/DCM, FL rt 3 h | 72 : 7 |
| 4 | DCCA 1 mol/[NBu₄]Br₃ 2 mol/DCM, FL rt 4 h | 81 : 7 |
| 5 | DCCA 1 mol/[NBu₄]Br₃ 2 mol/DCM, FL rt 19 h | 67 : 6 |

| | | |
|---|---|---|
| ^{a} All quantities in mole/mole of lauric acid. | | |

### EXAMPLE 13

### Halo-de-carboxylation of 4-chlorophenylacetic acid

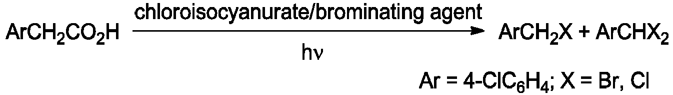

A mixture of 4-chlorophenylacetic acid (1 mmol), chloroisocyanurate, brominating agent and solvent (6 mL) was stirred under fluorescent room light irradiation (FL). An aliquot of the reaction mixture washed with 1 M aq Na₂SO₃, dried over Na₂SO₄, and filtered through short neutral silica gel pad. The yields of 4-chlorobenzyl bromide and chloride, and 4-chlorobenzal bromide were determined by gas chromatography (GC) using 1,2,4-trichlorobenzene as internal standard. The results are presented in Table 9.

**Table 9. Halo-de-carboxylation of 4-chlorophenylacetic acid ArCH₂CO₂H (Ar = 4-ClC₆H₄)^{a}**

| **entry** | **Reaction conditions** | **GC yields, % ArCH₂Br/ArCH₂Cl/ ArCHBr₂** |
|---|---|---|
| 1 | TCCA 1 mol/[NBu₄]Br₃ 1 mol/DCM, FL rt 1 h | 43 : 9 : 2 |
| 2 | TCCA 1 mol/[NBu₄]Br₃ 1 mol/DCM, FL rt 2 h | 42 : 14 : 4 |
| 3 | TCCA 1 mol/[NBu₄]Br₃ 1 mol/DCM, FL rt 2 h | 37 : 17 : 12 |
| 4 | DCCA 1 mol/[NBu₄]Br₃ 1 mol/DCM, FL rt 1 h | 75 : 5 : 0 |
| 5 | DCCA 1 mol/[NBu₄]Br₃ 1 mol/DCM, FL rt 2 h | 80 : 8 : 0 |
| 6 | DCCA 1 mol/[NBu₄]Br₃ 1 mol/DCM, FL rt 3 h | 74 : 11 : 3 |
| 7 | DCCA 1 mol/[NBu₄]Br₃ 1 mol/DCM, FL rt 6 h | 57 : 21 : 4 |
| 8 | DCCA 1 mol/[NBu₄]Br₃ 1 mol/DCM, FL rt 27 h | 22 : 42 : 7 |
| 9 | DCCA 1 mol/[NBu₄]Br₃ 2 mol/DCM, FL rt 0.5 h | 47 : 1 : 0 |
| 10 | DCCA 1 mol/[NBu₄]Br₃ 2 mol/DCM, FL rt 1 h | 80 : 2 : 1 |
| 11 | DCCA 1 mol/[NBu₄]Br₃ 2 mol/DCM, FL rt 2 h | 86 : 6 : 1 |

| | | |
|---|---|---|
| ^{a} All quantities in mole/mole of 4-chlorophenylacetic acid. | | |

### EXAMPLE 14

### Comparative Examples.

### A. Attempts to chlorodecarboxylate arenecarboxylic acids with N-chlorosuccinimide (NCS) under heterolytic reaction conditions disclosed in IN803DEL1999

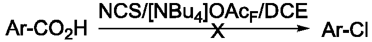

The reactions were provided under fluorescent room lighting (FL).

### Example 14A-1. An attempt to chlorodecarboxylate benzoic acid using tetrabutylammonium trifluororacetate as catalyst

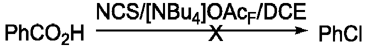

A mixture of benzoic acid (0.44 g, 3.60 mmol), *N*-chlorosuccinimide NCS (0.46 g, 3.44 mmol), tetrabutylammonium trifluororacetate [NBu₄]OAc_{F} (0.24 g, 0.67 mmol) and 1,2-dichloroethane DCE (6 mL) was stirred at rt for 24 h. The reaction mixture was washed with 1 M aq Na₂SO₃ (2 × 10 mL), dried over Na₂SO₄, and filtered through short neutral alumina pad. The obtained filtrate did not contain chlorobenzene (GC data, 1-chlro-2-fluorobenzene was used as internal standard).

### Example 14A-2. An attempt to chlorodecarboxylate p-toluic acid using tetrabutylammonium trifluororacetate as catalyst

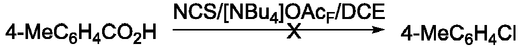

A mixture of *p*-toluic acid (0.48 g, 3.52 mmol), *N*-chlorosuccinimide NCS (0.46 g, 3.44 mmol), tetrabutylammonium trifluororacetate [NBu₄]OAc_{F} (0.24 g, 0.67 mmol) and 1,2-dichloroethane DCE (6 mL) was stirred at rt for 24 h. The reaction mixture was washed with 1 M aq Na₂SO₃ (2 × 10 mL), dried over Na₂SO₄, and filtered through short neutral alumina pad. The obtained filtrate did not contain *p*-chlorotoluene (GC data, *o-*dichlorobenzene was used as internal standard).

### Example 14A-3. An attempt to chlorodecarboxylate p-anisic acid using tetrabutylammonium trifluororacetate as catalyst

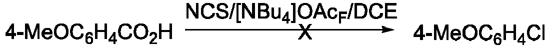

A mixture of *p*-anisic acid (0.52 g, 3.42 mmol), *N*-chlorosuccinimide NCS (0.46 g, 3.44 mmol), tetrabutylammonium trifluororacetate [NBu₄]OAc_{F} (0.24 g, 0.67 mmol) and 1,2-dichloroethane DCE (6 mL) was stirred at rt for 24 h. The reaction mixture was washed with 1 M aq Na₂SO₃ (2 × 10 mL), dried over Na₂SO₄, and filtered through short neutral alumina pad. The obtained filtrate did not contain *p*-chloroanisol (GC data, 1,2,4-trichlorobenzene was used as internal standard).

### B. Attempts to chlorodecarboxylate arenecarboxylic acids with N-chlorosuccinimide (NCS) under heterolytic reaction conditions disclosed in J. Dispersion Sci. Technol. 2007, v. 28, 613

### Example 14B-1: An attempt to chlorodecarboxylate 2-bromobenzoic acid using cetyltrimethylammonium bromide as catalyst

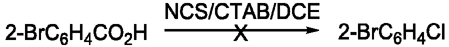

A mixture of 2-bromobenzoic acid (0.20 g, 1.0 mmol), *N*-chlorosuccinimide NCS (0.20 g, 1.5 mmol), cetyltrimethylammonium bromide CTAB (1.82 g, 5.0 mmol) and 1,2-dichloroethane DCE (10 mL) was stirred in dark under reflux conditions for 3 h. The cooled reaction mixture was washed with 1 M aq Na₂SO₃ (2 x 5 mL), dried over Na₂SO₄, filtered through short neutral alumina pad and concentrated in vacuo to give 2-chloroethyl 2-bromobenzoate 2-BrC₆H₄CO₂(CH₂)₂Cl (0.27 g, 100%). ¹H NMR: δ 7.85 (d, *J* = 7 Hz, 1H), 7.64 (d, *J* = 7 Hz, 1H), 7.38-7.28 (m, 2H) 4.56 (t, *J* = 6 Hz, 2H), 3.80 (t, *J* = 6 Hz, 2H) ppm.

### Example 14B-2: An attempt to chlorodecarboxylate 2-bromobenzoic acid using sodium dodecyl sulfate as catalyst

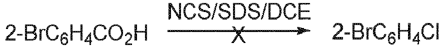

A mixture of 2-bromobenzoic acid (0.20 g, 1 mmol), N-chlorosuccinimide NCS (0.20 g, 1.5 mmol), sodium dodecyl sulfate SDS (1.44 g, 5.0 mmol) and 1,2-dichloroethane DCE (10 mL) was stirred in the dark under reflux conditions for 3 h. After it was cooled, the reaction mixture was washed with 1 M aq Na₂SO₃ (2 x 5 mL), dried over Na₂SO₄, filtered through short neutral alumina pad and concentrated in vacuo. The residue (15 mg) does not contain 1-bromo-2-chlorobenzene as determined by ¹H NMR.

While certain features of the invention have been illustrated and described herein, many modifications, substitutions, changes, and equivalents will now occur to those of ordinary skill in the art.

## Claims

1. A process for the preparation of organic halide of formula **(1A)** from a carboxylic acid of formula **(2A)** represented by scheme 1: 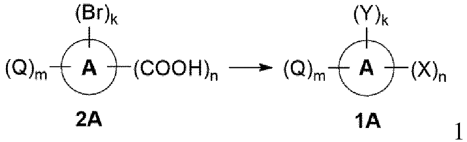 said process comprises radical halo-de-carboxylation reaction comprising reacting carboxylic acid **(2A)** with a chloroisocyanurate and a brominating agent to yield organic halide **(1A);** wherein
said chloroisocyanurate is trichloroisocyanuric acid, dichloroisocyanuric acid, or any combination thereof;
**A** is arene, alkane, cycloalkane or saturated heterocycle;
**n** is an integer greater than or equal to 1;
**X** is Cl or Br; wherein if n > 1, then X may be the same or different;
**k** is an integer greater than or equal to 0;
**Y** is Cl or Br; wherein if k > 1, then Y may be the same or different;
**m** is an integer greater than or equal to 0;
each **Q** is independently F, Cl, Br, R¹, acyl, C(O)R¹, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R¹, N(R¹)SO₂R¹, N(acyl)₂, N[C(O)R¹]SO₂R¹, N[C(O)R¹]₂, CF₃; or any two vicinal Q substituents are joined to form a 5- or 6-membered substituted or unsubstituted, saturated or unsaturated carbocyclic or heterocyclic ring;
wherein each R¹ is independently aryl, alkyl, cycloalkyl or heterocyclyl, wherein said R¹ is optionally substituted by one or more substituents of R²;
wherein each R² is independently F, Cl, Br, COOH, acyl, aryl, alkyl, cycloalkyl or heterocyclyl; wherein if either one of R² in (**2A**) is carboxylic group COOH, then the respective R² in (**1A**) is Br or Cl;
wherein the position of said X, Y and Q in said structure of formula (**1A**) correspond to the same position of said COOH, Br and Q, respectively in said structure of formula **(2A).**

2. A radiation-sensitive composition comprising a carboxylic acid **(2A)** 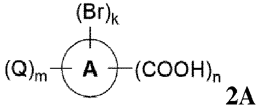 a chloroisocyanurate and a brominating agent which generates organic halide (**1A**) 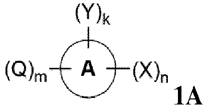 upon electromagnetic irradiation,
wherein
said chloroisocyanurate is trichloroisocyanuric acid, dichloroisocyanuric acid, or any combination thereof;
**A** is arene, branched alkane, cycloalkane or saturated heterocycle;
**n** is an integer greater than or equal to 1;
**X** is Cl or Br; wherein if n > 1, then X may be the same or different;
**k** is an integer greater than or equal to 0;
**Y** is Cl or Br; wherein if k > 1, then Y may be the same or different;
**m** is an integer greater than or equal to 0;
each **Q** is independently F, Cl, Br, R¹, acyl, C(O)R¹, C(O)OR¹, C(O)OMe, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R¹, N(R¹)SO₂R¹, N(acyl)₂, N[C(O)R¹]SO₂R¹, N[C(O)R¹]₂, CF₃; or any two vicinal Q substituents are joined to form a 5- or 6-membered substituted or unsubstituted, saturated or unsaturated carbocyclic or heterocyclic ring;
wherein each R¹ is independently aryl, alkyl, cycloalkyl or heterocyclyl, wherein R¹ is optionally substituted by one or more substituents of R² ;
wherein each R² is independently F, Cl, Br, COOH, acyl, aryl, alkyl, cycloalkyl or heterocyclyl;
wherein if either one of R² in (**2A**) is a carboxylic group COOH, then the respective R² in (**1A**) is Br or Cl;
wherein the position of said Br and Q in said structure of formula (**1A**) correspond to the same position of said COOH and Q, respectively in said structure of formula **(2A).**

3. The process of claim 1, or the composition of claim 2, wherein A is benzene.

4. The process of claim 1, or the composition of claim 2, wherein said organic halide of formula (**1A**) is a mixture of organic halide products of formula (**1A**) with different X groups; if k is different than 0, then the organic halide products optionally have also different Y group.

5. The process of claim 1, or the composition of claim 2, wherein X and Y in said organic halide (**1A**) are bromine atoms when **A** is an alkane, cycloalkane or saturated heterocycle.

6. The process of claim 1, or the composition of claim 2, wherein said organic halide is haloarene of formula (**1B**) 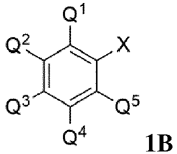 and said carboxylic acid is arenecarboxylic acid of formula (**2B**) 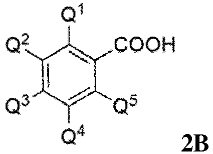 wherein
X is Cl or Br;
each of Q¹, Q², Q³, Q⁴, and Q⁵, are independently selected from: H, F, Cl, Br, R¹, COOH, acyl, C(O)R¹, acetyl, benzoyl, C(O)OR¹, C(O)Cl, C(O)N(R¹)₂, CN, SO₂R¹, SO₃R¹, NO₂, N(R¹)₃⁺, OR¹, OCF₃, O-acyl, OC(O)R¹, OSO₂R¹, SR¹, S-acyl, SC(O)R¹, N(R¹)acyl, N(R¹)C(O)R², N(R¹)SO₂R¹, N(acyl)₂, N[C(O)R¹]SO₂R¹, N[C(O)R¹]₂, CF₃; or any two of Q¹ and Q², Q² and Q³, Q³ and Q⁴, or Q⁴ and Q⁵, are joined to form a 5- or 6-membered substituted or unsubstituted, saturated or unsaturated carbocyclic or heterocyclic ring;
wherein each R¹ is independently aryl, alkyl, cycloalkyl or heterocyclyl; wherein R¹ is optionally substituted by one or more substituents of R² ;
wherein each R² is independently F, Cl, Br, COOH, acyl, aryl, alkyl, cycloalkyl or heterocyclyl;
wherein if either one of Q¹, Q², Q³, Q⁴, Q⁵, and/or R² in (**2B**) is carboxylic group COOH, then the respective Q¹, Q², Q³, Q⁴, Q⁵, and/or R² in (**1B**) is Cl or Br;
wherein if either one of Q¹, Q², Q³, Q⁴, Q⁵, and/or R² in (**2B**) is bromine (Br) then the respective Q¹, Q², Q³, Q⁴, Q⁵, and/or R² in (**1B**) is Cl or Br.

7. The process or the composition of claim 6, wherein if either one of Q¹, Q², Q³, Q⁴, Q⁵ is a nitro group, then X is Br, or wherein if none of the substituents Q¹, Q², Q³, Q⁴ and Q⁵ are a nitro group, then X is Cl.

8. The process or the composition of claim 7, wherein if none of the substituents Q¹, Q², Q³, Q⁴ and Q⁵ are a nitro group and if either one of Q¹, Q², Q³, Q⁴, or Q⁵ in arenecarboxylic acid **(2B)** is Br then the respective Q¹, Q², Q³, Q⁴, or Q⁵ in haloarene (**1B**) is Cl.

9. The process or the composition of claim 6, wherein at least one of Q¹, Q², Q³, Q⁴, and/or Q⁵ is F, Cl, Br, CF₃, CCl₃, CN, COOH, C(O)OMe, NO₂, OCF₃, and/or any two of Q¹ and Q², Q² and Q³, Q³ and Q⁴, or Q⁴ and Q⁵, are joined to form a dihydrofuran-2,5-dione or pyrrolidine-2,5-dione ring.

10. The process of claim 1, or the composition of claim 2, wherein said brominating agent is Br₂ (bromine), a salt containing bromide or polybromide anion and organic or inorganic cation; or any combination thereof.

11. The process or the composition of claim 10, wherein said polybromide anion is an ion of formula
[Brₚ]^{q-}
where **p** is an integer of at least 3 and **q** is an integer of at least 1 and not more than p/2.

12. The process of claim 1, or the composition of claim 2, wherein the molar ratio between said brominating agent: (each carboxylic group of the carboxylic acid of formula **(2A)**) is between 0.1 and 4.

13. The process of claim 1, wherein in order to accelerate the radical reaction the reaction mixture is subjected to electromagnetic irradiation or is conducted in the presence of radical initiator.

14. The process of claim 13, or the composition of claim 2, wherein said electromagnetic irradiation is microwave, infrared, ultraviolet, or visible light irradiation or any combination thereof.

## Patentansprüche

1. Prozess zur Herstellung von organischem Halogenid der Formel (**1A**) aus einer Carbonsäure der Formel **(2A),** der durch das Reaktionsschema 1 dargestellt ist: 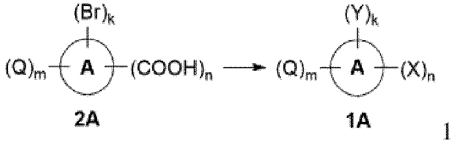 wobei der Prozess eine radikale Halo-Decarboxylierungsreaktion umfassend ein Umsetzen von Carbonsäure (**2A**) mit einem Chlorisocyanurat und einem Bromierungsmittel, um organisches Halogenid (**1A**) zu erhalten, umfasst;
wobei
das Chlorisocyanurat Trichlorisocyanursäure, Dichlorisocyanursäure oder eine Kombination davon ist;
**A** Aren, Alkan, Cycloalkan oder ein gesättigter Heterocyclus ist;
**n** eine ganze Zahl größer oder gleich 1 ist;
**X** Cl oder Br ist; wobei, wenn n > 1, dann kann X gleich oder verschieden sein;
**k** eine ganze Zahl größer oder gleich 0 ist;
**Y** Cl oder Br ist; wobei, wenn k > 1, dann kann Y gleich oder verschieden sein;
**m** eine ganze Zahl größer oder gleich 0 ist;
**Q** jeweils unabhängig F, Cl, Br, R¹, Acyl, C(O)R¹, C(O)OR¹, C(O)Cl, C(O)N(R¹)₂, CN, SO₂R¹, SO₃R¹, NO₂, N(R¹)₃₊, OR¹, OCF₃, O-Acyl, OC(O)R¹, OSO₂R¹, SR¹, S-Acyl, SC(O)R¹, N(R¹)Acyl, N(R¹)C(O)R¹, N(R¹)SO₂R¹, N(Acyl)₂, N[C(O)R¹]SO₂R¹, NFC(O)R¹]₂, CF₃ ist; oder zwei beliebige vic. Q-Substituenten werden zur Bildung eines 5- oder 6-gliedrigen substituierten oder unsubstituierten, gesättigten oder ungesättigten carbocyclischen oder heterocyclischen Rings miteinander verbunden;
wobei R¹ jeweils unabhängig Aryl, Alkyl, Cycloalkyl oder Heterocyclyl ist, wobei R¹ wahlweise durch einen oder mehrere Substituenten von R² substituiert ist;
wobei R² jeweils unabhängig F, Cl, Br, COOH, Acyl, Aryl, Alkyl, Cycloalkyl oder Heterocyclyl ist; wobei, falls der eine oder andere R² in (**2A**) eine Carbongruppe COOH ist, dann ist der resp. R² in (**1A**) Br oder Cl;
wobei die Position von X, Y und Q in der Struktur der Formel (**1A**) derselben Position von COOH, Br und Q resp. in der Struktur der Formel (**2A**) entspricht.

2. Strahlungsempfindliche Zusammensetzung, umfassend eine Carbonsäure (**2A**) 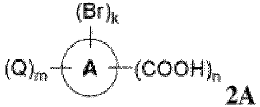 ein Chlorisocyanurat und ein Bromierungsmittel, das organisches Halogenid (**1A**) erzeugt 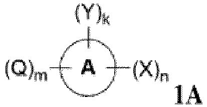 wenn es elektromagnetischer Bestrahlung ausgesetzt ist,
wobei
das Chlorisocyanurat Trichlorisocyanursäure, Dichlorisocyanursäure oder eine Kombination davon ist;
**A** Aren, verzweigtes Alkan, Cycloalkan oder ein gesättigter Heterocyclus ist;
**n** eine ganze Zahl größer oder gleich 1 ist;
**X** Cl oder Br ist; wobei gilt, falls n > 1, dann kann X gleich oder verschieden sein;
**k** eine ganze Zahl größer oder gleich 0 ist;
**Y** Cl oder Br ist; wobei, falls k > 1, dann kann Y gleich oder verschieden sein;
**m** eine ganze Zahl größer oder gleich 0 ist;
**Q** jeweils unabhängig F, Cl, Br, R¹, Acyl, C(O)R¹, C(O)OR¹, C(O)OMe, C(O)Cl, C(O)N(R¹)₂, CN, SO₂R¹, SO₃R¹, NO₂, N(R¹)₃₊, OR¹, OCF₃, O-Acyl, OC(O)R¹, OSO₂R¹, SR¹, S-Acyl, SC(O)R¹, N(R¹)Acyl, N(R¹)C(O)R¹, N(R¹)SO₂R¹, N(Acyl)₂, N[C(O)R¹]SO₂R¹, NFC(O)R¹]₂, CF₃ ist; oder zwei beliebige vic. Q-Substituenten werden zur Bildung eines 5- oder 6-gliedrigen substituierten oder unsubstituierten, gesättigten oder ungesättigten carbocyclischen oder heterocyclischen Rings miteinander verbunden;
wobei R¹ jeweils unabhängig Aryl, Alkyl, Cycloalkyl oder Heterocyclyl ist, wobei R¹ wahlweise mit einem oder mehreren Substituenten von R² substituiert ist;
wobei R² jeweils unabhängig F, Cl, Br, COOH, Acyl, Aryl, Alkyl, Cycloalkyl oder Heterocyclyl ist;
wobei, falls der eine oder andere von R² in (**2A**) eine Carbonsäuregruppe COOH ist, dann ist der resp. R² in (**1A**) Br oder Cl;
wobei die Position von Br und Q in der Struktur der Formel (**1A**) derselben Position von COOH und Q resp. in der Struktur der Formel (**2A**) entspricht.

3. Prozess nach Anspruch 1 oder Zusammensetzung nach Anspruch 2, wobei A Benzol ist.

4. Prozess nach Anspruch 1 oder Zusammensetzung nach Anspruch 2, wobei das organische Halogenid der Formel (**1A**) ein Gemisch organischer Halogenidprodukte der Formel (**1A**) mit verschiedenen X-Gruppen ist; wenn k von 0 verschieden ist, dann können, die organischen Halogenidprodukte wahlweise auch eine verschiedene Y-Gruppe haben.

5. Prozess nach Anspruch 1 oder Zusammensetzung nach Anspruch 2, wobei X und Y in dem organischen Halogenid (**1A**) Bromatome sind, wenn A ein Alkan, Cycloalkan oder ein gesättigter Heterocyclus ist.

6. Prozess nach Anspruch 1 oder Zusammensetzung nach Anspruch 2, wobei das organische Halogenid Haloaren der Formel (**1B**) ist 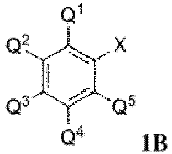 und die Carbonsäure Arencarbonsäure der Formel (**2B**) ist 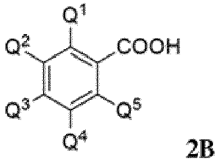 wobei
X Cl oder Br ist;
Q¹, Q², Q³, Q⁴ und Q⁵ jeweils unabhängig unter Folgendem ausgewählt sind: H, F, Cl, Br, R¹, COOH, Acyl, C(O)R¹, Acetyl, Benzoyl, C(O)OR¹, C(O)Cl, C(O)N(R¹)₂, CN, SO₂R¹, SO₃R¹, NO₂, N(R¹)₃₊, OR¹, OCF₃, O-Acyl, OC(O)R¹, OSO₂R¹, SR¹, S-Acyl, SC(O)R¹, N(R¹)Acyl, N(R¹)C(O)R², N(R¹)SO₂R¹, N(Acyl)₂, N[C(O)R¹]SO₂R¹, N[C(O)R¹]₂, CF₃; oder beliebig zwei von Q¹ and Q², Q² und Q³, Q³ und Q⁴ oder Q⁴ und Q⁵ miteinander verbunden werden, um einen 5- oder 6-gliedrigen substituierten oder unsubstituierten, gesättigten oder ungesättigten carbocyclischen oder heterocyclischen Ring zu bilden;
wobei R¹ jeweils unabhängig Aryl, Alkyl, Cycloalkyl oder Heterocyclyl ist; wobei R¹ wahlweise mit einem oder mehreren Substituenten von R² substituiert ist;
wobei R² jeweils unabhängig F, Cl, Br, COOH, Acyl, Aryl, Alkyl, Cycloalkyl oder Heterocyclyl ist;
wobei, falls der eine oder andere von Q¹, Q², Q³, Q⁴, Q⁵ und/oder R² in (**2B**) eine Carbongruppe COOH ist, dann ist der resp. Q¹, Q², Q², Q⁴, Q⁵ und/oder R² in (**1B**) Cl oder Br;
wobei, falls der eine oder andere von Q¹, Q², Q³, Q⁴, Q⁵ und/oder R² in (**2B**) Brom (Br) ist, dann ist die resp. Q¹, Q², Q², Q⁴, Q⁵ und/oder R² in (**1B**) Cl oder Br.

7. Prozess oder Zusammensetzung nach Anspruch 6, wobei gilt, wenn der eine oder andere von Q¹, Q², Q³, Q⁴, Q⁵ eine Nitrogruppe ist, dann ist X Br, oder wobei, wenn keiner der Substituenten Q¹, Q², Q³, Q⁴ und Q⁵ eine Nitrogruppe ist, dann ist X Cl.

8. Prozess oder Zusammensetzung nach Anspruch 7, wobei, wenn keiner der Substituenten Q¹, Q², Q³, Q⁴ und Q⁵ eine Nitrogruppe ist und wenn der eine oder andere von Q¹, Q², Q³, Q⁴ oder Q⁵ in Arencarbonsäure (**2B**) Br ist, dann ist der resp. Q¹, Q², Q², Q⁴ oder Q⁵ in Haloaren (**1B**) Cl.

9. Prozess oder Zusammensetzung nach Anspruch 6, wobei mindestens einer von Q¹, Q², Q³, Q⁴ und/oder Q⁵ F, Cl, Br, CF₃, CCl₃, CN, COOH, C(O)OMe, NO₂, OCF₃ ist und/oder beliebige zwei von Q¹ und Q², Q² und Q³, Q³ und Q⁴ oder Q⁴ und Q⁵ miteinander verbunden werden, um einen Dihydrofuran-2,5-dion- oder Pyrrolidin-2,5-dionring zu bilden.

10. Prozess nach Anspruch 1 oder Zusammensetzung nach Anspruch 2, wobei das Bromierungsmittel Br₂ (Brom), ein Bromid oder Polybromidanionen und organische oder anorganische Kationen enthaltendes Salz ist; oder eine beliebige Kombination davon.

11. Prozess oder Zusammensetzung nach Anspruch 10, wobei das Polybromidanion ein Ion der Formel ist
[Brₚ]^{q-}
wobei **p** eine ganze Zahl von mindestens 3 ist und **q** eine ganze Zahl von mindestens 1 und nicht mehr als p/2 ist.

12. Prozess nach Anspruch 1 oder Zusammensetzung nach Anspruch 2, wobei das Molverhältnis von Bromierungsmittel / (jeder Carboxygruppe der Carbonsäure der Formel **(2A)**) zwischen 0,1 und 4 ist.

13. Prozess nach Anspruch 1, wobei das Gemisch elektromagnetischer Bestrahlung unterworfen wird, um die Umsetzung der Radikale des Reaktionsgemisches zu beschleunigen oder die Umsetzung bei Anwesenheit eines Radikalinitiators durchgeführt wird.

14. Prozess nach Anspruch 13 oder Zusammensetzung nach Anspruch 2, wobei die elektromagnetische Bestrahlung mit Mikrowellen, Infrarot, Ultraviolett oder sichtbarem Licht oder einer Kombination davon erfolgt.

## Revendications

1. Procédé de préparation d'halogénure organique de formule **(1A)** à partir d'un acide carboxylique de formule **(2A)** représenté par le schéma 1 : 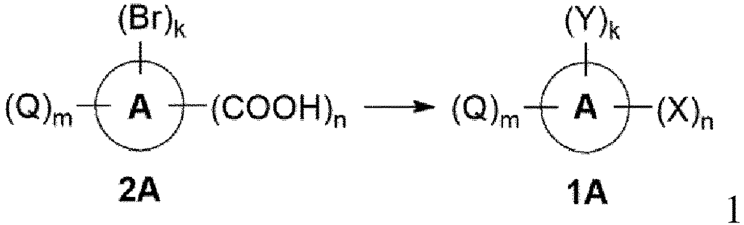 ledit procédé comprend une réaction d'halogéno-dé-carboxylation radicalaire comprenant la réaction d'acide carboxylique **(2A)** avec un chloroisocyanurate et un agent de bromation pour donner un halogénure organique **(1A)** ; dans lequel
ledit chloroisocyanurate est l'acide trichloroisocyanurique, l'acide dichloroisocyanurique ou toute combinaison de ces derniers ;
A est un arène, un alcane, un cycloalcane ou un hétérocycle saturé ;
n est un entier supérieur ou égal à 1 ;
X est Cl ou Br ; dans lequel si n>1, alors X peut être identique ou différent ;
k est un entier supérieur ou égal à 0 ;
Y est Cl ou Br ; dans lequel si k>1, alors Y peut être identique ou différent ;
m est un entier supérieur ou égal à 0 ;
chaque Q est indépendamment F, Cl, Br, R¹, acyle, C(O)R¹, C(O)OR¹, C(O)Cl, C(O)N(R¹)₂, CN, SO₂R¹, SO₃R¹, NO2, N(R¹)₃₊, OR¹, OCF₃, O-acyle, OC(O)R¹, OSO₂R¹, SR¹, S-acyle, SC(O)R¹, N(R¹)acyle, N(R¹)C(O)R¹, N(R¹)SO₂R¹, N(acyle)₂, N[C(O)R¹]SO₁R¹, N[C(O)R¹]₂, CF₃ ; ou deux substituants Q vicinaux quelconques sont joints pour former un noyau carbocyclique ou hétérocyclique à 5 ou 6 chaînons substitué ou non substitué, saturé ou insaturé ;
dans lequel chaque R¹ est indépendamment aryle, alkyle, cycloalkyle ou hétérocyclyle, où ledit R¹ est facultativement substitué par un ou plusieurs substituants de R² ;
dans lequel chaque R² est indépendamment F, Cl, Br, COOH, acyle, aryle, alkyle, cycloalkyle ou hétérocyclyle ; dans lequel si l'un des R² dans **(2A)** est un groupe carboxylique COOH, alors le R² respectif dans **(1A)** est Br ou Cl ;
dans lequel la position desdits X, Y et Q dans ladite structure de formule **(1A)** correspond à la même position desdits COOH, Br et Q, respectivement dans ladite structure de formule **(2A).**

2. Composition sensible aux radiations comprenant un acide carboxylique **(2A)** 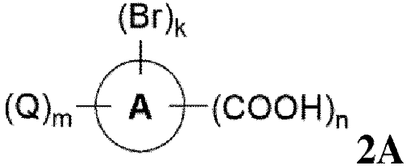 un chloroisocyanurate et un agent de bromation qui génère un halogénure organique **(1A)** 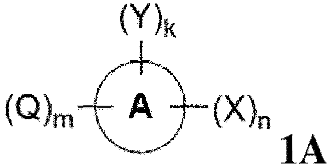 après irradiation électromagnétique, dans laquelle
ledit chloroisocyanurate est l'acide trichloroisocyanurique, l'acide dichloroisocyanurique ou toute combinaison de ces derniers ;
**A** est un arène, un alcane ramifié, un cycloalcane ou un hétérocycle saturé ;
**n** est un entier supérieur ou égal à 1 ;
**X** est Cl ou Br ; dans lequel si n>1, alors X peut être identique ou différent ;
**k** est un entier supérieur ou égal à 0 ;
**Y** est Cl ou Br ; dans lequel si k>1, alors Y peut être identique ou différent ;
**m** est un entier supérieur ou égal à 0 ;
chaque **Q** est indépendamment F, Cl, Br, R¹, acyle, C(O)R¹, C(O)OR¹, C(O)Cl, C(O)N(R¹)₂, CN, SO₂R¹, SO₃R¹, NO₂, N(R¹)₃₊, OR¹, OCF₃, O-acyle, OC(O)R¹, OSO₂R¹, SR¹, S-acyle, SC(O)R¹, N(R¹)acyle, N(R¹)C(O)R¹, N(R¹)SO₁R¹, N(acyle)₂, N[C(O)R¹]SO₂R¹, N[C(O)R¹]₂, CF₃ ; ou deux substituants Q vicinaux quelconques sont joints pour former un noyau carbocyclique ou hétérocyclique à 5 ou 6 chaînons substitué ou non substitué, saturé ou insaturé;
dans lequel chaque R¹ est indépendamment aryle, alkyle, cycloalkyle ou hétérocyclyle, où R¹ est facultativement substitué par un ou plusieurs substituants de R² ;
dans laquelle chaque R² est indépendamment F, Cl, Br, COOH, acyle, aryle, alkyle, cycloalkyle ou hétérocyclyle ;
dans laquelle si l'un ou l'autre de R² dans **(2A)** est un groupe carboxylique COOH, alors le R² respectif dans **(1A)** est Br ou Cl ;
dans laquelle la position desdits Br et Q dans ladite structure de formule **(1A)** correspond à la même position desdits COOH et Q, respectivement dans ladite structure de formule **(2A).**

3. Procédé selon la revendication 1 ou composition selon la revendication 2, dans lequel A est le benzène.

4. Procédé selon la revendication 1, ou composition selon la revendication 2, dans lequel ledit halogénure organique de formule **(1A)** est un mélange de produits halogénures organiques de formule **(1A)** avec différents groupes X ; si k est différent de 0, alors les produits halogénures organiques ont éventuellement également un groupe Y différent.

5. Procédé selon la revendication 1, ou composition selon la revendication 2, dans lequel X et Y dans ledit halogénure organique **(1A)** sont des atomes de brome lorsque A est un alcane, un cycloalcane ou un hétérocycle saturé.

6. Procédé selon la revendication 1, ou composition selon la revendication 2, dans lequel ledit halogénure organique est l'haloarène de formule **(1B)** 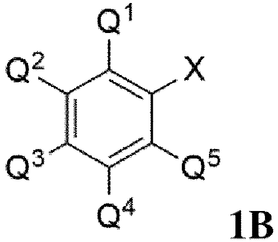 et ledit acide carboxylique est l'acide arènecarboxylique de formule **(2B)** 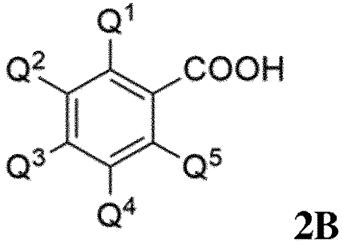 dans lequel
X est Cl ou Br ;
chacun des Q1, Q2, Q3, Q4 et Q5, est indépendamment sélectionné parmi : H, F, Cl, Br, R1, COOH, acyle, C(O)R¹, acétyle, benzoyle, C(O)OR¹, C(O)Cl, C(O)N(R¹)₂, CN, SO₂R¹, SO₃R¹, NO₂, N(R¹)₃₊, OR¹, OCF₃, O-acyle, OC(O)R¹, OC(O)R¹, OSO₂R¹, SR¹, S-acyle, SC(O)R¹, N(R¹)acyle, N(R¹)C(O)R², N(R¹)SO₁R¹, N(acyle)₂, N[C(O)R¹]SO₂R¹, N[C(O)R¹]₂, CF₃ ; ou deux quelconques parmi Q1 et Q2, Q2 et Q3, Q3 et Q4, ou Q4 et Q5, sont joints pour former un noyau carbocyclique ou hétérocyclique à 5 ou 6 chaînons substitué ou non, saturé ou insaturé ;
dans lequel chaque R¹ est indépendamment aryle, alkyle, cycloalkyle ou hétérocyclyle ; dans lequel R¹ est facultativement substitué par un ou plusieurs substituants de R² ;
dans lequel chaque R² est indépendamment F, Cl, Br, COOH, acyle, aryle, alkyle, cycloalkyle ou hétérocyclyle ;
dans lequel si l'un des Q¹, Q², Q³, Q⁴, Q⁵ et/ou R² dans **(2B)** est un groupe carboxylique COOH, alors les Q¹, Q², Q³, Q⁴, Q⁵ et/ou R² respectifs dans **(1B)** sont Cl ou Br ;
dans lequel si l'un des Q¹, Q², Q³, Q⁴, Q⁵ et/ou R² dans **(2B)** est le brome (Br), alors les Q¹, Q², Q³, Q⁴, Q⁵ et/ou R² respectifs dans **(1B)** sont Cl ou Br.

7. Procédé ou composition selon la revendication 6, dans lequel si l'un des Q¹, Q², Q³, Q⁴, Q⁵ est un groupe nitro, alors X est Br, ou dans lequel si aucun des substituants Q¹, Q², Q³, Q⁴ et Q⁵ n'est un groupe nitro, alors X est Cl.

8. Procédé ou composition selon la revendication 7, dans lequel si aucun des substituants Q¹, Q², Q³, Q⁴ et Q⁵ sont un groupe nitro et si l'un des Q¹, Q², Q³, Q⁴ ou Q⁵ dans l'acide arénecarboxylique **(2B)** est Br, alors les Q¹, Q², Q³, Q⁴ ou Q⁵ respectifs dans l'haloarène **(1B)** est Cl.

9. Procédé ou composition selon la revendication 6, dans lequel au moins l'un des Q¹, Q², Q³, Q⁴ et/ou Q⁵ est F, Cl, Br, CF₃, CCl₃, CN, COOH, C(O)OMe, NO₂, OCF₃, et/ou deux quelconques parmi Q¹ et Q², Q² et Q³, Q³ et Q⁴, ou Q⁴ et Q⁵, sont joints pour former un cycle dihydrofuran-2,5-dione ou pyrrolidine-2,5-dione.

10. Procédé selon la revendication 1, ou composition selon la revendication 2, dans lequel ledit agent bromant est Br₂ (brome), un sel contenant un anion bromure ou polybromure et un cation organique ou inorganique ; ou toute combinaison de ceux-ci.

11. Procédé ou composition selon la revendication 10, dans lequel ledit anion polybromide est un ion de formule
[Brₚ]^{q-}
où **p** est un entier d'au moins 3 et **q** est un entier d'au moins 1 et d'au plus p/2.

12. Procédé selon la revendication 1, ou composition selon la revendication 2, dans lequel le rapport molaire entre ledit agent bromant : (chaque groupe carboxylique de l'acide carboxylique de formula **(2A))** est compris entre 0,1 et 4.

13. Procédé selon la revendication 1, dans lequel, pour accélérer la réaction radicalaire, le mélange réactionnel est soumis à une irradiation électromagnétique ou est conduit en présence d'un initiateur radicalaire.

14. Procédé selon la revendication 13, ou composition selon la revendication 2, dans lequel ladite irradiation électromagnétique est une irradiation par micro-ondes, infrarouge, ultraviolette ou lumière visible ou toute combinaison de ces dernières.
